# EUROPEAN PATENT SPECIFICATION

(11) **EP 3 627 153 B1**
(45) Date of publication and mention of the grant of the patent: **07.10.2020**
(21) Application number: 19192403.4
(22) Date of filing: 03.06.2016
(51) Int. Cl.: G01N 33/50

(54) **A METHOD OF ASSESSING PROTEIN MODIFICATION STATUS AND IDENTIFYING BIOMARKERS LINKED TO CELL SIGNALING PATHWAYS**
VERFAHREN ZUR BEURTEILUNG DES PROTEINMODIFIZIERUNGSSTATUS UND ZUR IDENTIFIZIERUNG VON MIT ZELLSIGNALWEGEN VERBUNDENEN BIOMARKERN
PROCÉDÉ D'ÉVALUATION D'ÉTAT DE MODIFICATION DE PROTÉINE ET D'IDENTIFICATION DE BIOMARQUEURS LIÉS À DES CHEMINS DE SIGNALISATION DE CELLULES

(30) Priority: 05.06.2015 GB 201509961
(43) Date of publication of application: 25.03.2020
(62) Divisional of application: 16732321.1
(73) Proprietor: Queen Mary University of London, London E1 4NS (GB)
(72) Inventor: CUTILLAS, Pedro Rodriguez, London EC1M 6BQ (GB); WILKES, Edmund, London W6 8RF (GB)
(74) Representative: Newton, Michael Jonathan

(56) References cited:
- EP-A1- 2 124 051
- C. PAN ET AL: "Global Effects of Kinase Inhibitors on Signaling Networks Revealed by Quantitative Phosphoproteomics", MOLECULAR & CELLULAR PROTEOMICS, vol. 8, no. 12, 3 August 2009 (2009-08-03) , pages 2796-2808, XP055292647, US ISSN: 1535-9476, DOI: 10.1074/mcp.M900285-MCP200
- UWE RIX ET AL: "Chemical proteomic profiles of the BCR-ABL inhibitors imatinib, nilotinib, and dasatinib reveal novel kinase and nonkinase targets", BLOOD, vol. 110, no. 12, 1 December 2007 (2007-12-01), pages 4055-4063, XP055292654, DOI: 10.1182/blood-2007-
- DATABASE MEDLINE [Online] US NATIONAL LIBRARY OF MEDICINE (NLM), BETHESDA, MD, US; December 2013 (2013-12), VOJVODIC MILIANA ET AL: "A phosphoproteomics approach to identify candidate kinase inhibitor pathway targets in lymphoma-like primary cell lines.", XP008181060, Database accession no. NLM23701117 & VOJVODIC MILIANA ET AL: "A phosphoproteomics approach to identify candidate kinase inhibitor pathway targets in lymphoma-like primary cell lines.", CURRENT DRUG DISCOVERY TECHNOLOGIES DEC 2013, vol. 10, no. 4, December 2013 (2013-12), pages 283-304, ISSN: 1875-6220
- D. S. KIRKPATRICK ET AL: "Phosphoproteomic characterization of DNA damage response in melanoma cells following MEK/PI3K dual inhibition", PROCEEDINGS OF THE NATIONAL ACADEMY OF SCIENCES, vol. 110, no. 48, 11 November 2013 (2013-11-11), pages 19426-19431, XP055292529, US ISSN: 0027-8424, DOI: 10.1073/pnas.1309473110 & Donald S Kirkpatrick ET AL: "Correction for "Phosphoproteomic characterization of DNA dam- age response in melanoma cells following MEK/PI3K dual in- hibition," by", Proc Natl Acad Sci, 26 November 2013 (2013-11-26), pages 19426-19431, XP055292534, Retrieved from the Internet: URL:http://www.pnas.org/content/111/1/562. 2.full.pdf
- EDMUND H. WILKES ET AL: "Empirical inference of circuitry and plasticity in a kinase signaling network", PROCEEDINGS OF THE NATIONAL ACADEMY OF SCIENCES, vol. 112, no. 25, 9 June 2015 (2015-06-09) , pages 7719-7724, XP055292512, US ISSN: 0027-8424, DOI: 10.1073/pnas.1423344112 & Reagents: "SI Appendix to "Empirical inference of circuitry and plasticity in a kinase signaling network"", , 9 June 2015 (2015-06-09), XP055292540, Retrieved from the Internet: URL:http://www.pnas.org/content/suppl/2015 /06/08/1423344112.DCSupplemental/pnas.1423 344112.sapp.pdf [retrieved on 2016-08-01]

## Description

### Field of the Invention

The present invention relates to a method of creating a dataset of modification sites which can be used to prepare a database of protein modification sites, for example phosphorylation sites that are linked to signaling pathways. This is useful because it provides information on which sites are modified in response to modulation by particular drugs, such as inhibitors. A database containing the data can then be used as an input to other methods for analysing data such as phosphoproteomic data and can be used to identify markers of kinases that are inhibited by particular compounds or are associated with particular phenotypes.

### Background to the Invention

Cell signaling pathways form complex networks of biochemical reactions that integrate and decode extracellular signals into appropriate responses. The reconstruction of these networks, and systematic analyses of their properties, is important in the advancement of our molecular understanding of disease at the systems level. The topology and plasticity of cell signaling networks play major roles in fundamental and disease physiology. Attempts to characterize such molecular organization have relied on inference algorithms that obtain information on protein interactions and post-translational modification (PTMs) from the literature (Kholodenko BN, et al. (2002) Proc. Natl. Acad. Sci. U.S.A. 99(20):12841-12846; Mukherjee S & Speed TP (2008) Proc. Natl. Acad. Sci. U.S.A. 105(38):14313-14318; Prill RJ, et al (2011) Sci. Signal 4(189):mr7). The accuracy of network reconstruction using such models is limited by the availability of data (Prill RJ, *et al, supra),* and by the fact that signaling events are often cell-type specific. As a result, although they can provide insightful data, models that derive network topologies from studies that have utilized different cell types and organisms result in composite or averaged networks, which, critically, do not always reflect network structure in specific cell-types, at specific stages of cell development, or under defined physiological conditions (Prill RJ, *et al, supra).*

Pan et al (2009) Molecular & Cellular Proteomics 8(12): 2796 - 2808 describes global effects of kinase inhibitors on signaling networks revealed by quantitative phosphoproteomics. Rix et al (2007) Blood 110(12): 4055 - 4063 describes chemical proteomic profiles of the BCR-ABL inhibitors imatinib, nilotinib, and dasatinib reveal novel kinase and nonkinase targets. Vojvodic et al (2013) Current Drug Discovery Technologies 10(4): 283 - 304 describes a phosphoproteomics approach to identify candidate kinase inhibitor pathway targets in lymphoma-like primary cell lines. Kirkpatrick et al (2013) Proc. Natl. Acad. Sci. U.S.A. 110(48): 19426 - 19431 describes phosphoproteomic characterization of DNA damage response in melanoma cells following MEK/PI3K dual inhibition. EP2124051 describes a method for rapid generation of phosphorylation profiles, the detection of *in vivo* phosphorylation sites of kinases and phosphatases and their use as diagnostic markers in cells, tissues and body fluids.

The maturation of phosphoproteomics techniques based on mass spectrometry (MS) is now allowing the simultaneous quantification of several thousands of phosphorylation sites per experiment, and approaches to derive kinase activity from these large-scale phosphoproteomics datasets have been reported (Linding R, et al. (2007) Cell 129(7):1415-1426; Carlson SM, et al. (2011) Sci. Signal. 4(196); Bensimon A, et al (2012) Annu. Rev. Biochem. 81:379-405; Casado P, et al. (2013) Sci. Signal. 6(268):rs6).

One such approach, named Kinase Substrate Enrichment Analysis (KSEA), is based on the premise that, as each phosphorylation site is the result of a kinase's catalytic activity, phosphoproteomic profiling provides a means by which to capture and measure the activities of all kinases expressed in the system under investigation (Casado P, *et al, supra).* KSEA is a technique to systematically infer protein kinase pathway activation from MS-based phosphoproteomics data and is the subject of the inventors' patent application published as WO 2013/132075. However, the KSEA method relies on a compilation of phosphorylation sites known from the literature and from databases such as PhosphoSite (Hornbeck et al, Proteomics 4, 1551 (Jun, 2004)) and PhosphoElm (Dinkel et al, Nucleic Acids Res 39, D261 (Jan, 2011)).

There is a need in the art for a method to analyse signaling networks without the prior knowledge of phosphorylation sites on particular proteins.

### Summary of the Invention

The present inventors first used MS-based phosphoproteomics to define a kinase signaling network by systematically identifying phosphorylation sites downstream of kinases targeted by small-molecule kinase inhibitors of the PI3K/Akt/mTOR and MEK-ERK signaling axes. These two ubiquitous pathways form a network that regulates growth factor, antigen and insulin signaling whilst also being deregulated in most cancers. The inventors then measured the activity and plasticity of different routes within this experimentally-defined kinase signaling network in cells chronically treated with small-molecule inhibitors of kinases in these pathways. It was found that remodeling of kinase networks in resistant cells produced patterns of signaling activity linked to their evolved phenotypes.

The methods developed by the present inventors can be used to create a dataset of phosphorylation sites that are linked to signaling pathways. This is useful because it provides information on which sites are phosphorylated or dephosphorylated in response to modulation by particular drugs, such as inhibitors. A database containing the data can then be used as an input to other methods for analyzing phosphoproteomic data, for example the KSEA method previously developed by the present inventors, and can be used to identify markers of kinases that are inhibited by particular compounds. The methods are not limited to the analysis of phosphorylation sites, but can be used to create a dataset of protein modification sites that can be detected using MS-based methods.

Aspects of the invention for which protection is sought are defined in the claims.

According to a first aspect, the invention provides an *in vitro* method of creating a dataset of modification sites which are affected in the same way by modulators of the same protein modifying enzyme, and are therefore indicative of the activity of the protein modifying enzyme that is affected by the modulators, comprising identifying and/or quantifying modification sites on modified peptides in a first sample which has been treated with a first modulator of a protein modifying enzyme and a second sample which has been treated with a second modulator of the same protein modifying enzyme using mass spectrometry (MS); and grouping modification sites on modified peptides from the first sample which has been treated with the first modulator of a protein modifying enzyme and modification sites on modified peptides from the second sample which has been treated with the second modulator of the same protein modifying enzyme into a single group, according to the effect of said first and second modulators of said protein modifying enzyme on said modification sites, wherein the modification sites on modified peptides from a first sample and modification sites on modified peptides from a second sample are placed into a single group based on the modification sites being affected in the same way by the first and second modulators of the protein modifying enzyme, and wherein said first and second modulators of said protein modifying enzyme are different.

Said modification sites may be selected from the group consisting of phosphorylation sites, acetylation sites, glycosylation sites, methylation sites and lipidation sites. Said first sample and said second sample may be from either:
(a) a bodily fluid, optionally urine or blood; or
(b) a tissue, optionally cancer tissue, optionally a tumor.

Said protein modifying enzyme may be selected from the group consisting of a protein kinase, protein phosphatase, protein glycosyltransferase, protein acetyltransferase, protein methyltransferase and protein palmitoyltransferase. Said modulator of a protein modifying enzyme may be:
(a) a small molecule, RNAi, therapeutic peptide, or antibody; and/or
(b) an inhibitor of a protein modifying enzyme.
Said inhibitor of a protein modifying enzyme may be a kinase inhibitor. Said kinase inhibitor may be an inhibitor of a kinase selected from the group consisting of Akt, CAMK2, EGFR, ERK, MEK, mTOR, p70S6K, PI3K, PKC, and ROCK.

Said effect of said first and second modulators of said protein modifying enzyme on said modification site may be a reduction or increase in abundance of said modification site compared to a control sample.

According to a second aspect, the invention provides a method of preparing a database, comprising creating a dataset of modification sites according to a method of the first aspect, and compiling said dataset into a database. Said database may further comprise information on the identities of proteins containing the modification sites, the type of modification, the type of sample in which the modification site is present and/or the modulator that increases or decreases the modification at the modification site.

According to a third aspect, the invention provides an *in vitro* method for diagnosing a disease comprising:
(a) Creating a test dataset of modification sites by:
   i. Treating one or more test samples from a subject with one or more modulators of a protein modifying enzyme,
   ii. Identifying and/or quantifying modification sites on modified peptides in the one or more test samples,
   iii. Grouping modification sites on modified peptides from the one or more test samples into a single group, thereby creating the test dataset of modification sites, and
   iv. optionally compiling the test dataset into a test database,
(b) Comparing the test dataset with a dataset of modification sites created according to the method of the first aspect of the invention or comparing the test database a database prepared according to the second aspect of the invention,
(c) Finding a significant difference between the test dataset or test database and the control dataset or control database,
(d) Diagnosing that the subject has the disease if a significant difference between the dataset or database is found in step (c).

The disease may be cancer.

According to a fourth aspect, the invention provides a method of identifying one or more biomarkers for the activity of a protein modifying enzyme comprising creating a dataset of modification sites according to the method of the first aspect of the invention or preparing a database according to the second aspect of the invention, wherein the modification sites in the single group are identified as the one or more biomarkers for the activity of a protein modifying enzyme.

According to a fifth aspect, the invention provides an *in vitro* method of determining that a test substance is a modulator of a protein modifying enzyme comprising treating a test sample with the test substance, identifying and/or quantifying modification sites on modified peptides in the test sample, creating a test dataset of modification sites from the modification sites identified and/or quantified in the test sample, further comprising:
(a) Comparing the test dataset with a dataset of modification sites created according to the method of the first aspect of the invention and/or
(b) Compiling said test dataset into a test database and comparing the test database with a database prepared according the second aspect of the invention.

### Detailed Description of the Invention

Described herein is a method of creating a dataset of modification sites by analyzing modification sites on modified peptides from a first and a second sample. Most proteins and peptides are modified in some way by the addition or removal of functional groups and such modifications are effected by protein modifying enzymes. Protein and peptide modifications that can be detected by mass spectrometry (MS) include phosphorylation, glycosylation, acetylation, methylation and lipidation. Such modifications are examples of post-translational modifications, and have various biological roles in the cell. The modification sites may therefore be sites of post-translational modifications. For example, the modification sites may be sites may be sites of phosphorylation, glycosylation, acetylation, methylation and lipidation. The modification sites are typically protein and/or peptide modification sites. A modification site may be one or more amino acid residues of a peptide or protein to which a functional group such as a phosphate group is added to the peptide or protein. Alternative functional groups include carbohydrates, acetyl groups, methyl groups and lipids. A "modified peptide" is defined herein as a peptide which has been modified by the addition or removal of a functional group. A "protein modifying enzyme" is defined herein as an enzyme which catalyses a reaction involving the addition or removal of a functional group to a protein or peptide.

A "peptide" as defined herein is a short amino acid sequence and includes oligopeptides and polypeptides. Typically, such peptides are between about 5 and 30 amino acids long, for example from 6 or 7 to 25, 26 or 27 amino acids, from 8, 9 or 10 to 20 amino acids, from 11 or 12 to 18 amino acids or from 14 to 16 amino acids, for example 15 amino acids. However, shorter and longer peptides, such as between about 2 and about 50, for example from about 3 to about 35 or 40 or from about 4 to about 45 amino acids can also be used. Typically, the peptide is suitable for mass spectrometric analysis, that is the length of the peptide is such that the peptide is suitable for mass spectrometric analysis. The length of the peptide that can be analysed is limited by the ability of the mass spectrometer to sequence such long peptides. In certain cases polypeptides of up to 300 amino acids can be analysed, for example from 50 to 250 amino acids, from 100 to 200 amino acids or from 150 to 175 amino acids.

The method can be applied to the analysis of any post-translational modifications that can be detected using MS-based methods. Such modifications include those that are mediated by enzymes include protein kinases, protein phosphatases, protein glycosyltransferases, protein acetyltransferases, protein methyltransferases and protein palmitoyltransferases. The activity of these enzymes results in phosphorylation, dephosphorylation, acetylation, glycosylation, methylation and lipidation of protein or peptide substrates respectively. All of these protein/peptide modifications can be detected by mass spectrometry.

Accordingly, the modified peptides for use in the method are typically phosphorylated peptides, dephosphorylated peptides, acetylated peptides, glycosylated peptides, methylated peptides and/or lipidated peptides. The modified peptides contain one or more amino acid with a post-translational modification, which is specific to the protein modifying enzyme that causes the modification. Such modified amino acids are referred to herein as "modification sites".

The modified peptides for use in the method may be phosphorylated peptides. Phosphorylated peptides contain one or more amino acid which is phosphorylated (i.e. a phosphate (PO₄) group has been added to that amino acid). Such phosphorylated amino acids are referred to herein as "phosphorylation sites". In relation to this, the term "phosphoprotein" is used herein to refer to a phosphorylated protein and the term "phosphopeptide" is used herein to refer to a phosphorylated peptide.

Human protein kinases can be divided into a number of groups including AGC kinases, for example protein kinase A (PKA), protein kinase B (PKB) (also known as Akt), protein kinase C (PKC) and protein kinase G (PKG); tyrosine kinases such as receptor tyrosine kinases (for example the epidermal growth factor receptor (EGFR)); tyrosine-kinase like kinases; calcium/calmodulin-dependent protein kinases (for example CAMK2); the casein kinase 1 group; CMGC group, for example CDK, MAPK (such as extracellular-signal-related kinases (ERK), mitogen-activated protein kinase kinases (for example MEK)), GSK3 and CLK kinases; and STE, the homologues of yeast Sterile 7, Sterile 11, and Sterile 20 kinases. Other kinases include mTOR (mammalian target of rapamycin), p70S6 kinase and rho-associated protein kinase (ROCK), all of which are serine/threonine protein kinases, and phosphatidylinositol-4,5-bisphosphate 3-kinase (PI3K).

Protein phosphatases include protein phosphatase 2 (PP2 or PP2A).

The method involves grouping modification sites on modified peptides from a first sample and modification sites on modified peptides from a second sample into a single group. The first and second samples used in the methods can be any samples which contain peptides. The sample is typically a biological sample and can thus be any type of sample obtained from a biological source, for example a sample obtained from a human, animal, plant or bacterium. The samples used may be obtained from human and non-human sources.

The samples used in the methods can be from any species of interest. Typically, the samples are from a human or animal. The animal is typically a mammal, for example a rodent such as a mouse, rat or guinea pig, or an ungulate such as a cow, sheep or goat. The animal is alternatively a bird, such as a chicken, a fish, such as a zebra fish, a nematode, such as the worm *Caenorhabditis elegans,* or an insect, such as the fruit fly *Drosophila melanogaster.* The samples used in the methods can also be from other life-forms such as bacteria and yeast. The samples used in the methods are typically samples from an experimentally important species of bacterium such as *Escherichia coli, Salmonella enterica, Streptococcus pneumoniae* or *Staphylococcus aureus,* or of yeast such as the baker's yeast *Saccharomyces cerevisiae* or the fission yeast *Schizosaccharomyces pombe.* The samples used in the methods can alternatively be from a plant or fungus or a virus.

Typically, the biological sample is derived from a human, and can be, for example, a sample of a bodily fluid such as urine or blood, or another tissue. Typically, the biological sample is a cell from a cell line or a tissue, typically a primary tissue. For example, the sample can be a tissue from a human or animal. The human or animal can be healthy or diseased. The tissue may be cancer tissue. For example, the sample may be from a tumor. Alternatively, the sample can be a cell line derived from healthy or diseased human or animal cells.

The first and second samples used in the method typically are or include cells from cell lines, for example a cancer cell line such as a breast cancer cell line, for example an MCF7 cell line. Many cancer cell lines are known in the art and are listed online, for example in the Broad-Novartis Cancer Cell Line Encyclopedia (CCLE) at http://www.broadinstitute.org/ccle/home

The first and second samples are typically from the same cell line but may be different. The first and second samples may be derived from the same source. For example, the first and second samples may both be from a single individual. The first and second samples may be from the same tissue. The first and second samples may be from the same bodily fluid.

A sample may be referred to herein as a "test sample" in order to distinguish the sample from another sample used in a method. For example, a sample may be referred to as a test sample in order describe a method involving a comparison with a control sample.

The method is an *in vitro* method and therefore does not comprise the step of obtaining a sample from an organism such as an animal.

The modified peptides may be modified in the sample. The modified peptides may be modified *in vivo.* The modified peptides may be endogenous modified peptides.

In the present invention, the first and second samples have been treated with a modulator of a protein modifying enzyme prior to carrying out the method of the invention. In practice, this means that either the first and second samples themselves or the organism from which the first and second samples are obtained are treated with a modulator of a protein modifying enzyme prior to carrying out the method of the invention.

The method may also comprise the step of obtaining modified peptides from the first and/or second sample. For example, when the first and/or second sample is or includes a cell (for example from a cell line), the modified peptides can be obtained from the cell by lysing (splitting open) the cell, extracting proteins from the lysed cells and cleaving (breaking down or digesting) the proteins into peptides. The cells can be lysed using any suitable means known in the art, for example using physical methods such as mechanical lysis (for example using a Waring blender), liquid homogenization, sonication or manual lysis (for example using a pestle and mortar) or detergent-based methods such as CHAPS or Triton-X. Typically, the cells are lysed using a denaturing buffer such as a urea-based buffer. Protein cleavage or digestion can be carried out using any suitable agent known in the art and is typically carried out using a protease. Any suitable protease can be used. The protease is typically trypsin, chymotrypsin, Arg-C, pepsin, V8, Lys-C, Asp-C and/or AspN. Alternatively, the proteins can be cleaved chemically, for example using hydroxylamine, formic acid, cyanogen bromide, BNPS-skatole, 2-nitro-5-thiocyanobenzoic acid (NTCB) or any other suitable agent.

The protein modifying enzyme is typically selected from the group consisting of a protein kinase, protein phosphatase, protein glycosyltransferase, protein acetyltransferase, protein methyltransferase and protein palmitoyltransferase. In one embodiment, the protein modifying enzyme is a protein kinase.

The modulators of a protein modifying enzyme used to treat the first and second samples prior to carrying out the method are modulators of the same protein modifying enzyme, but the modulators used to treat the first and second samples are different, typically structurally different.

The modulator of a protein modifying enzyme is typically an exogenous chemical or drug, such as a small molecule (such as a small molecule inhibitor), RNAi, therapeutic peptide, or antibody.

The modulator of a protein modifying enzyme may modulate the activity of a protein modifying enzyme in any way. The modulator of a protein modifying enzyme may be an inhibitor of a protein modifying enzyme. The inhibitor of a protein modifying enzyme may be a kinase inhibitor. The modulator of a protein modifying enzyme may be an activator of a protein modifying enzyme. The inhibitor of a protein modifying enzyme may be a kinase activator.

Typical kinase inhibitors include inhibitors of Akt, such as Akt inhibitor VIII and MK-2206, inhibitors of CAMK2, such as KN-93 and KN-62, inhibitors of EGFR, such as PD-168393 and PD-153035, inhibitors of ERK, such as ERK inhibitor and ERK inhibitor II, inhibitors of MEK, such as GSK-1120212 and U0126, inhibitors of mTOR, such as KU-0063794 and Torin-1, inhibitors of p70S6K, such as PF-4708671 and DG2, inhibitors of PI3K, such as GDC-0941 and PI-103, inhibitors of PKC, such as Gö-6976 and BIM-1, and inhibitors of ROCK, such as H-1152 and Y-27632.

Other inhibitors of PI3K include PP2 and wortmannin. At least 80 kinase inhibitors are in different stages of clinical development (Zhang, J.; et al Nat Rev Cancer 2009, 9, (1), 28-39).

The inhibitor of a protein modifying enzyme may be a phosphatase inhibitor. Inhibitors of phosphatases include inhibitors of PP2A, such as okadaic acid and cantharidic acid.

The method comprises grouping modification sites on modified peptides from a first sample which has been treated with a first modulator of a protein modifying enzyme and modification sites on modified peptides from a second sample which has been treated with a second modulator of the same protein modifying enzyme into a single group. By "grouping" it is meant that modification sites such as phosphorylation sites are placed into a group or set. In the method, modification sites on modified peptides from a first sample and modification sites on modified peptides from a second sample are placed into a single group based on the effect of the first and second modulators of the protein modifying enzyme on the modification sites. In other words, the modification sites on modified peptides from a first sample and modification sites on modified peptides from a second sample are placed into a single group based on the modification sites being affected in the same way by the first and second modulators of the protein modifying enzyme.

For example, the effect of the first and second modulators of the protein modifying enzyme on the modification sites can be a reduction or increase in abundance (frequency) of a particular modification site relative to a control sample. In this context, the modification sites on modified peptides from a first sample and modification sites on modified peptides from a second sample are placed into a single group based on a similar reduction or increase in abundance of a particular modification site in response to the first and the second modulators of the protein modifying enzyme, compared to a control sample. The reduction or increase in abundance is typically a statistically significant reduction or increase and can be calculated using any suitable statistical method, for example as described in the Example herein.

The end result of the method is a dataset of modification sites which are affected in the same way by modulators of the same protein modifying enzyme, and are therefore indicative of the activity of the protein modifying enzyme that is affected by the modulator. The selection of modification sites inhibited by different (and typically structurally distinct) modulators targeting the same protein modifying enzyme results in datasets enriched in modification sites specific to the intended protein modifying enzyme. If a particular modification site on a modified peptide is reduced in abundance by at least two different modulators of the same protein modifying enzyme, then it represents a read-out of the protein modifying enzyme that is actually affected by the modulator. Such modification sites are referred to herein as "compound-target activity markers" (CTAMs). Accordingly, such modification sites may be considered to be biomarkers for the activity of a protein modifying enzyme.

The method may involve grouping modification sites on modified peptides from more than two samples, for example 3, 4, 5, 6, 7, 8, 9, 10 or even more samples, each of which has been treated with a different modulator of the same protein modifying enzyme. In this context, the method involves grouping modification sites on modified peptides from all of the samples into a single group according to the effect of each of the modulators of the same protein modifying enzyme on the modified peptides, as described herein. For example, when the method involves 3 samples, the method involves grouping modification sites on modified peptides from a first sample which has been treated with a first modulator of a protein modifying enzyme, modification sites on modified peptides from a second sample which has been treated with a second modulator of the same protein modifying enzyme and modification sites on modified peptides from a third sample which has been treated with a third modulator of the same protein modifying enzyme into a single group, according to the effect of said first, second and third modulators of said protein modifying enzyme on said modification sites, wherein said first, second and third modulators of said protein modifying enzyme are different. For example, the effect of the first, second and third modulators of the protein modifying enzyme on the modification sites can be a reduction or increase in abundance (number) of a particular modification site relative to a control sample. In this context, the modification sites on modified peptides from a first sample, modification sites on modified peptides from a second sample and modification sites on modified peptides from a third sample are placed into a single group based on a similar reduction or increase in abundance of a particular modification site in response to the first, second and third modulators of the protein modifying enzyme, compared to a control sample. Where more than two samples are used, there can be a single control sample against which each of the test samples are compared.

The outcome of the method is a dataset of modification sites (for example phosphorylation sites) that are affected by modulators of protein modifying enzymes, such as inhibitors of protein kinases. The knowledge of such modification sites enables the linking of inhibitors to the activities of kinases and the phenotype of cells. The knowledge of such modification sites also provides information regarding the circuitry of signaling pathways and when monitored in other experiments such modification sites can also reveal plasticity of signaling pathways; that is, how these change upon determined experimental conditions or during disease progression. The method can also be used to identify new markers or biomarkers, and thus provide information on the activity of kinases and other protein modifying enzymes targeted by specific inhibitors.

The method, the dataset created according to the method and/or the database prepared according to the method can also be used in a method for diagnosing a disease. The disease may be cancer. The first sample and the second sample may be from the subject. The first sample and the second sample may be from a tumor of the subject. The first sample and the second sample may be from different stages of disease progression. The method of diagnosis may comprise administering one or more modulators of a protein modifying enzyme to the first sample and the second sample *in vitro.* The method may enable identification of the therapeutic resistance mechanisms relevant to each individual patient. The method of diagnosis may further comprise assessing cellular responses to the one or more modulators of the protein modifying enzyme. The cellular responses may, for example, be proliferation or cell viability. The cellular responses may be correlated with the dataset and/or the database. The method of diagnosis may comprise comparison of the dataset or database obtained from the samples from the subject with one or more control samples. Typically the control samples will be from healthy tissue or a healthy subject. The control samples may be from healthy or diseased tissue or from a healthy of diseased subject. The comparison with control samples may indicate that one or more protein modifying enzymes are deregulated in the subject relative to the control. The method of diagnosis may further comprise identification of therapeutic targets relevant to the subject. The method of diagnosis may further comprise determining an effective modulator of a protein modifying enzyme.

The method, the dataset created according to the method and/or the database prepared according to the method can also be used in a method of determining that a test substance is a modulator of a protein modifying enzyme. Accordingly, method, the dataset created according to the method and/or the database prepared according to the method can also be used in a method of screening. The method of screening may be a method of screening test substances to determine whether the test substance is a modulator of a protein modifying enzyme. The test substance may be an exogenous chemical or drug, such as a small molecule (such as a small molecule inhibitor), RNAi, a therapeutic peptide, or an antibody. The test substance may be a novel substance or a known substance. The test substance may be a composition.

Determining that a test substance is a modulator of a protein modifying enzyme may therefore involve determining that the test substance is an inhibitor of a protein modifying enzyme. Alternatively, the test substance may be an activator of the protein modifying enzyme. For example, the test substance may be identified as a kinase inhibitor or a kinase activator.

The method is based on the analysis of modification sites on modified peptides identified using MS-based techniques. Accordingly, the modification sites on modified peptides from a first sample and from a second sample which are grouped in the first step of the method are typically identified and/or quantified using MS-based techniques. The method therefore includes a step of identifying and/or quantifying modification sites on modified peptides in a first sample and/or a second sample using mass spectrometry (MS), prior to the step of grouping the modification sites on modified peptides from a first sample and from a second sample into a single group, as described herein.

Identification and quantification of modification sites on modified peptides can be carried out using any suitable method. Typically, quantification can be carried out by any method involving mass spectrometry (MS), such as liquid chromatography-mass spectrometry (LC-MS). The LC-MS or LC-MS/MS is typically label-free MS but techniques that use isotope labelling as the basis for quantification can also be used as the basis for the analysis.

In the methods, quantification of a protein modification such as phosphorylation is typically carried out using the TIQUAS (targeted and in-depth quantification of signalling) technique, as described in WO 2010/119261 (International patent application no. PCT/GB2010/000770), as well as in Casado P, et al. (2013) Genome Biol. 14(4):R37 and Montoya A, et al. (2011) Methods 54(4):370-378. This technique allows for sensitive, rapid and comprehensive quantification of modified peptides. The method can, in one simple assay, simultaneously measure the amounts of thousands of phosphorylation sites on proteins. As set out in WO 2010/119261, the TIQUAS technique can also be used to quantify modified peptides other than phosphorylated peptides. In fact, the TIQUAS technique can be used to quantify peptides which contain any modifications which can be detected by mass spectrometry.

In this context, the step of quantifying modification sites on modified peptides using mass spectrometry (MS) prior to the grouping step is carried out using a method comprising the following steps:
(a) obtaining peptides from a sample;
(b) adding reference modified peptides to the peptides obtained in step (a) to produce a mixture of peptides and reference modified peptides;
(c) carrying out mass spectrometry (MS) on said mixture of peptides and reference modified peptides to obtain data relating to the peptides in the sample; and
(d) comparing the data relating to the peptides in the sample with data in a database of modified peptides using a computer programme;
wherein the database of modified peptides is compiled by a method comprising:
i obtaining peptides from a sample;
ii enriching modified peptides from the peptides obtained in step i;
iii carrying out liquid chromatography-tandem mass spectrometry (LC-MS/MS) on the enriched modified peptides obtained in step ii;
iv comparing the modified peptides detected in step iii to a known reference database in order to identify the modified peptides; and
v compiling data relating to the modified peptides identified in step iv into a database.

Where the protein modifying enzyme is a protein kinase and the modification is phosphorylation, the step of quantifying modification sites on modified peptides using mass spectrometry (MS) prior to the grouping step may be carried out using a method comprising the following steps:
(a) obtaining phosphorylated peptides from a sample;
(b) adding reference phosphorylated peptides to the peptides obtained in step (a) to produce a mixture of phosphorylated peptides and reference phosphorylated peptides;
(c) carrying out mass spectrometry (MS) on said mixture of phosphorylated peptides and reference phosphorylated peptides to obtain data relating to the phosphorylated peptides in the sample; and
(d) comparing the data relating to the phosphorylated peptides in the sample with data in a database of phosphorylated peptides using a computer programme;
wherein the database of phosphorylated peptides is compiled by a method comprising:
i obtaining peptides from a sample;
ii enriching phosphorylated peptides from the peptides obtained in step i;
iii carrying out liquid chromatography-tandem mass spectrometry (LC-MS/MS) on the enriched phosphorylated peptides obtained in step ii;
iv comparing the phosphorylated peptides detected in step iii to a known reference database in order to identify the phosphorylated peptides; and
v compiling data relating to the phosphorylated peptides identified in step iv into a database.

In relation to this method, the work "peptide" is used interchangeably with the word "polypeptide".

Step (a) of this method involves obtaining peptides from a sample. Peptides can be obtained from the sample using any suitable method known in the art. Step (a) may comprise:
(1) lysing cells in the sample;
(2) extracting the proteins from the lysed cells obtained in step (1); and
(3) cleaving said proteins into peptides.

In step (1), the cells in the sample are lysed, or split open. The cells can be lysed using any suitable means known in the art, for example using physical methods such as mechanical lysis (for example using a Waring blender), liquid homogenization, sonication or manual lysis (for example using a pestle and mortar) or detergent-based methods such as CHAPS or Triton-X. Typically, the cells are lysed using a denaturing buffer such as a urea-based buffer.

In step (2), proteins are extracted from the lysed cells obtained in step (1). In other words, the proteins are separated from the other components of the lysed cells.

In step (3), the proteins from the lysed cells are cleaved into peptides. In other words, the proteins are broken down into shorter peptides. Protein breakdown is also commonly referred to as digestion. Protein cleavage can be carried out using any suitable agent known in the art.

Protein cleavage or digestion is typically carried out using a protease. Any suitable protease can be used. The protease is typically trypsin, chymotrypsin, Arg-C, pepsin, V8, Lys-C, Asp-C and/or AspN. Alternatively, the proteins can be cleaved chemically, for example using hydroxylamine, formic acid, cyanogen bromide, BNPS-skatole, 2-nitro-5-thiocyanobenzoic acid (NTCB) or any other suitable agent.

In step (b) of this method, reference modified peptides (typically reference phosphorylated peptides) are added to the peptides obtained in step (a) to produce a mixture of peptides and reference modified peptides (typically reference phosphorylated peptides). Step (b) thus results in one mixture of peptides (including modified ones, typically phosphorylated ones) per sample. The reference modified peptides (typically reference phosphorylated peptides) are also referred to herein as "internal standards" (ISs). Typically, 5 to 10, for example 6 to 9 or 7 to 8, reference modified peptides (typically reference phosphorylated peptides) are added.

The reference modified peptides are typically reference phosphorylated peptides and are typically derived from a reference protein of defined nature and concentration, often referred to as an internal standard (IS) protein. ISs can be commercially available proteins, for example casein. Alternatively, ISs are synthesised specifically. In this context, reference phosphorylated peptides are typically synthesised with the same sequence as some of the phosphorylated peptides that it is desired to quantify but which are enriched in stable heavy isotopes of carbon and nitrogen. The peptides are typically synthesised using solid phase chemistry in which one amino acid is added at a time to form an amino acid chain or polypeptide. Typically, such peptides are enriched in ¹³C and ¹⁵N that substitute the common ¹²C and ¹⁴N. This enrichment results in the reference phosphorylated peptides being approximately 6 to 10 daltons heavier than the endogenous phosphorylated peptides with the same sequence so that they can be distinguished using a mass spectrometer.

When the protein modifying enzyme is a protein acetyltransferase and acetylated peptides are being quantified, the reference modified peptides are reference acetylated peptides. Such reference acetylated peptides are typically synthetic peptides containing acetylated amino acids.

The reference modified peptides (typically reference phosphorylated peptides) are typically added at a known amount in each of the samples to be compared. The signals of the endogenous modified peptides (typically phosphorylated peptides) are normalised to the signal of the reference modified peptides (typically reference phosphorylated peptides) in downstream analysis.

Step (b) of this method further comprises enriching modified peptides (typically phosphorylated peptides) from the mixture of peptides and reference modified peptides (typically reference phosphorylated peptides) obtained in step (b) to produce a mixture of enriched modified peptides (typically phosphorylated peptides). This additional step thus results in a single mixture of enriched modified peptides (typically phosphorylated peptides) per sample. In this context, step (c) thus comprises carrying out mass spectrometry (MS) on the mixture of enriched modified peptides (typically phosphorylated peptides) to obtain data relating to the peptides in the sample. In this context, step (b) typically results in a mixture of enriched modified peptides (typically phosphorylated peptides).

The step of enriching modified peptides (typically phosphorylated peptides) is typically carried out using chromatography. The chromatography may be immobilized metal ion affinity chromatography (IMAC), titanium dioxide (TiO₂) chromatography, and/or zirconium dioxide (ZrO₂) chromatography. Typically, the chromatography is IMAC and TiO₂ chromatography.

Alternatively, the step of enriching modified peptides (typically phosphorylated peptides) is carried out using antibody-based methods.

When the protein modifying enzyme is a protein kinase and the peptides being quantified are phosphorylated peptides, antibodies with affinity to phosphorylated amino acids such as tyrosine, threonine, serine or histidine may be linked (immobilised) to a solid matrix. Phosphorylated peptides are enriched by the ability of these antibodies to specifically bind phosphorylated peptides. Non-phosphorylated peptides are then washed away while phosphorylated peptides are retained on the antibody coated matrices. Elution of phosphorylated peptides from the immobilised antibody is typically carried out using low pH solvents or by any other suitable method that denatures the interaction between antibody and phosphorylated peptides.

When the protein modifying enzyme is a protein acetyltransferase and the peptides being quantified are acetylated peptides, acetylated peptides are enriched by the use of specific antibodies against acetylated amino acid residues. Such antibodies are linked to a solid matrix and then enriched by the ability of the antibodies to specifically bind acetylated amino acid residues. Non-acetylated peptides are then washed away while acetylated peptides are retained on the immobilised antibody.

In step (c) of this method, mass spectrometry (MS) is carried out on the mixture of peptides and reference modified peptides (typically reference phosphorylated peptides) obtained in step (b) to obtain data relating to the peptides in the sample. Typically, this data is in the form of an MS datafile for the sample. When step (b) of this method further comprises enriching modified peptides (typically phosphorylated peptides) from the mixture of peptides and reference modified peptides (typically reference phosphorylated peptides) obtained in step (b) to produce a mixture of enriched modified peptides (typically phosphorylated peptides), step (c) comprises carrying out mass spectrometry (MS) on said mixture of enriched modified peptides (typically phosphorylated peptides) to obtain data relating to the peptides in the sample, typically an MS datafile for the sample. Typically, the mass spectrometry is liquid chromatography-mass spectrometry (LC-MS). Step (c) thus typically results in an LC-MS datafile (one from each sample).

The data relating to the peptides in the sample typically comprises the mass to charge (m/z) ratio, charge (z) and/or relative retention time of the peptides.

In step (d) of this method, the data relating to the peptides in the sample (typically in the form of an MS datafile and more typically an LC-MS datafile) is compared with data in a database of modified peptides (typically phosphorylated peptides) using a computer programme. For example, the mass to charge (m/z) ratio, charge (z) and relative retention time of the peptides in the sample are compared with the mass to charge (m/z) ratio, charge (z) and relative retention time of the modified peptides (typically phosphorylated peptides) in the database. This enables the identification and quantification of each modified peptide (typically phosphorylated peptide) in the sample using the database of modified peptides (typically phosphorylated peptides).

Typically, the computer programme is the programme termed PESCAL (Cutillas, P. R.; Vanhaesebroeck, B. Mol Cell Proteomics 6(9), 1560-73, 2007). PESCAL constructs extracted ion chromatograms (XIC, i.e, an elution profile) for each of the modified peptides (typically phosphorylated peptides) present in the database across all the samples that are to be compared. This is done by centring the XIC on the m/z and retention time of the peptide previously identified to be modified (typically phosphorylated) (i.e, present in the database constructed in the first step of the procedure). PESCAL also considers the charge of the peptide to help in the correct assignment of identity. The program also calculates the peak height and area under the curve of each XIC. The data is normalised by dividing the intensity reading (peak areas or heights) of each modified peptides (typically phosphorylated peptide) that is being analysed by those of the reference modified peptides (typically reference phosphorylated peptides).

In this context, the database of modified peptides is compiled by a method comprising the following steps:
i obtaining peptides from a sample;
ii enriching modified peptides from the peptides obtained in step i;
iii carrying out liquid chromatography-tandem mass spectrometry (LC-MS/MS) on the enriched modified peptides obtained in step ii;
iv comparing the modified peptides detected in step iii to a known reference database in order to identify the modified peptides; and
v compiling data relating to the modified peptides identified in step iv into a database.

Step i involves obtaining peptides from a sample. Peptides can be obtained from the sample using any suitable method known in the art and as described herein. The sample of step i may be a third sample.

The sample is typically a biological sample and can thus be any type of sample obtained from a biological source, as described above. Typically, the sample is a cell line or a tissue.

Where the sample used in step i is a cell line, the sample may be treated with an inhibitor prior to carrying out step i. The inhibitor can be any suitable type of inhibitor. Typically, when phosphorylated peptides are being quantified, the inhibitor is a phosphatase inhibitor. Treatment with phosphatase inhibitors increases the stoichiometry of phosphorylation and results in a greater number of phosphorylated peptides that can be included in the database. In addition, methyl transferase or acetyl hydrolase inhibitors can be used when the purpose is to quantify methylated and acetylated peptides, respectively.

Step i may comprise:
(1) lysing cells in a sample;
(2) extracting the proteins from the lysed cells obtained in step (1); and
(3) cleaving said proteins into peptides.

These steps are as described above. However, step (3) is typically carried out using the same method as in step (a) described above.

In step ii, modified peptides (typically phosphorylated peptides) are enriched from the peptides obtained in step i. Step ii thus results in several fractions enriched in modified peptides (typically phosphorylated peptides).

The enrichment of modified peptides (typically phosphorylated peptides) in step ii is typically carried out using multidimensional chromatography. In one instance, the multidimensional chromatography is carried out using strong cation exchange high performance liquid chromatography (SCX-HPLC), immobilized metal ion affinity chromatography (IMAC) and titanium dioxide (TiO₂) chromatography. In another instance, the multidimensional chromatography is carried out using anion exchange high performance liquid chromatography (SAX-HPLC), immobilized metal ion affinity chromatography (IMAC) and titanium dioxide (TiO₂) chromatography. In these instances, the chromatographical techniques are carried out sequentially.

Alternatively, the enrichment of modified peptides (typically phosphorylated peptides) in step ii is carried out using antibody-based methods, as described above.

In step iii, liquid chromatography-tandem mass spectrometry (LC-MS/MS) is carried out on the enriched modified peptides (typically phosphorylated peptides) obtained in step ii.

In step iv, the modified peptides (typically phosphorylated peptides) detected in step iii are compared to a known reference database in order to identify the modified peptides (typically phosphorylated peptides). This step is typically carried out using a commercially available search engine, such as, but not restricted to, the MASCOT, ProteinProspector, or Sequest search engines.

In step v, data relating to the modified peptides (typically phosphorylated peptides) identified in step iv is compiled into a database. This database lists all the parameters needed for the quantification of phosphorylated peptides in subsequent biological experiments. Typically, the data relating to the modified peptides (typically phosphorylated peptides) includes identity of the modified peptides (typically phosphorylated peptide), mass to charge (m/z) ratio, charge and/or relative retention time. This allows data relating to the peptides in the sample, typically the mass to charge (m/z) ratio, charge (z) and relative retention time of the peptides in the sample, to be compared to the values for the modified peptides (typically phosphorylated peptides) in the database and thus allows the identification and quantification of the modified peptides (typically phosphorylated peptides) in the sample.

In this context, the compilation of the database does not need to be carried out simultaneously with the method. The compilation of the database can be carried out separately, in advance of the TIQUAS technique being used in the method to quantify the modification sites on a modified peptide in the sample.

The basis of the TIQUAS technique is the construction of a database of modified peptides (typically phosphorylated peptides) that can be detected and quantified by LC-MS. This database lists all the parameters needed for the quantification of modified peptides (typically phosphorylated peptides) in subsequent biological experiments including the identity of the modified peptide (typically phosphorylated peptide), mass to charge ratio (m/z), charge, and relative retention time. The database can be constructed by enriching modified peptides (typically phosphorylated peptides) using multidimensional chromatography (such as strong cation exchange, IMAC and TiO₂). Fractions of enriched modified peptides (typically phosphorylated peptides) can then be analysed by LC-MS/MS for identification of modified peptides (typically phosphorylated peptides).

The computer program named PESCAL (Cutillas and Vanhaesebroeck, Molecular & Cellular Proteomics 6, 1560-1573 (2007)) automates the quantification of each of the modified peptides (typically phosphorylated peptides) listed in the database in LC-MS runs of modified peptides (typically phosphorylated peptides) taken from biological experiments. For these biological experiments, proteins in cell lysates are digested using trypsin or other suitable proteases. Peptide (such as phosphopeptide) internal standards, which are reference modified peptides (typically reference phosphorylated peptides), are spiked at known amounts in all the samples to be compared. Modified peptides (typically phosphorylated peptides) in the resultant peptide mixture are enriched using a simple-to-perform IMAC or TiO₂ extraction step. Enriched modified peptides (typically phosphorylated peptides) are analysed in a single LC-MS run of typically but not restricted to about 120 minutes (total cycle). PESCAL then constructs extracted ion chromatograms (XIC, i.e, an elution profile) for each of the modified peptides (typically phosphorylated peptides) present in the database across all the samples that are to be compared. The program also calculates the peak height and area under the curve of each XIC. The data is normalised by dividing the intensity reading (peak areas or heights) of each modified peptide (typically phosphopeptide) analyte by those of the modified peptide (typically phosphopeptide) ISs.

As an alternative to using the TIQUAS technique, quantification of modifications such as phosphorylation can also be carried out using MS techniques that use isotope labels for quantification, such as metabolic labeling (e.g., stable isotope labeled amino acids in culture, (SILAC); Olsen, J.V. et al. Cell 127, 635-648 (2006)), and chemical derivatization (e.g., iTRAQ (Ross, P. L.; et al. Mol Cell Proteomics 2004, 3, (12), 1154-69), ICAT (Gygi, S.P. et al. Nat Biotechnol 17, 994-999 (1999)), TMT (Dayon L et al, Anal Chem. 2008 Apr 15;80(8):2921-31) techniques. In the methods, protein modifications can be quantified with LC-MS techniques that measure the intensities of the unfragmented ions or with LC-MS/MS techniques that measure the intensities of fragment ions (such as Selected Reaction Monitoring (SRM), also named multiple reaction monitoring (MRM)).

The present inventors have previously devised a technique to systematically infer protein kinase pathway activation from MS-based phosphoproteomics data. The technique is termed Kinase Substrate Enrichment Analysis (KSEA) and is described in (Casado P, *et al, supra)* as well as in the patent application published as WO 2013/132075.

As described in WO 2013/132075, the KSEA method is a method of quantifying the activity of a protein modifying enzyme in a sample, comprising:
(i) grouping modified peptides from a first sample and modified peptides from a second sample into a single group according to one of the following parameters:
   (a) modified peptides having a modification site that is modified by the same protein modifying enzyme; or
   (b) modified peptides having a modification site that is part of the same modification motif;
(ii) calculating enrichment of the modified peptides from the first sample compared to the modified peptides from the second sample in the group; and
(iii) calculating the statistical significance of said enrichment;
wherein a statistically significant enrichment is indicative of a protein modifying enzyme being activated in the first sample compared to the second sample.

In order to identify modified peptides having a modification site that is modified by the same protein modifying enzyme, the KSEA method relies on a compilation of phosphorylation sites known from the literature and from databases such as PhosphoSite (Hornbeck et al, Proteomics 4, 1551 (Jun, 2004)) and PhosphoElm (Dinkel et al, Nucleic Acids Res 39, D261 (Jan, 2011)). However, such databases are not comprehensive. The present invention allows the preparation of a database of protein modification sites which can be used as an input for the KSEA method without relying on such databases. In particular, the database which can be compiled using the dataset of modification sites produced by the method of the invention can be used as an input for step (i)(a) of KSEA method described above.

The present disclosure relates to a method of creating a dataset of modification sites, comprising grouping modified peptides from a first sample which has been treated with a first modulator of a protein modifying enzyme and modified peptides from a second sample which has been treated with a second modulator of the same protein modifying enzyme into a single group, according to the effect of said first and second modulators of said protein modifying enzyme on said modified peptides, wherein said first and second modulators of said protein modifying enzyme are different.

The method may comprise the steps of
(a) treating samples (for example cells from a cell line) with inhibitors against a panel of kinases;
(b) measuring the resulting changes in phosphorylation using MS-based phosphoproteomics;
(c) identifying phosphorylation sites that are significantly reduced in abundance by at least one kinase inhibitor;
(d) grouping phosphorylation sites based on behavior under treatment with inhibitors against same the kinase.

The result of this method is datasets enriched in phosphorylation sites specific to the intended kinase.

The dataset of modification sites produced according to the first aspect of the invention can then be used to prepare a database.

Accordingly, in a second aspect, the present invention provides a method of preparing a database, comprising creating a dataset of modification sites according to a method of the first aspect of the invention, and compiling said dataset into a database.

The database may also include information including, for example, the identities of proteins containing the modification sites, the type of modification, the type of sample in which the modification site is present and/or the modulator that increases or decreases the modification at the modification site. The database can then be used as an input to other methods for analysing data such as phosphoproteomic data, for example the KSEA method previously devised by the present inventors, and can be used to identify markers or biomarkers of kinases that are inhibited by particular compounds or are associated with particular phenotypes of cells in which the modification sites are found.

According to a third aspect, the invention provides an *in vitro* method for diagnosing a disease comprising:
(a) Creating a test dataset of modification sites by:
   i. Treating one or more test samples from a subject with one or more modulators of a protein modifying enzyme,
   ii. Identifying and/or quantifying modification sites on modified peptides in the one or more test samples,
   iii. Grouping modification sites on modified peptides from the one or more test samples into a single group, thereby creating the test dataset of modification sites, and
   iv. optionally compiling the test dataset into a test database,
(b) Comparing the test dataset with a dataset of modification sites created according to the first aspect of the invention or comparing the test database a database prepared according to the second aspect of the invention,
(c) Finding a significant difference between the test dataset or test database and the control dataset or control database,
(d) Diagnosing that the subject has the disease if a significant difference between the dataset or database is found in step (c).

Steps (b) and (c) may be performed by any suitable means. Step (b) may comprise the calculation of a reduction or increase in abundance of modification sites on modified peptides from the one or more test samples relative to the dataset of the modification sites created according to the first aspect of the invention or comparing the test database a database prepared according to the second aspect of the invention. The reduction or increase in abundance is typically a statistically significant reduction or increase and can be calculated using any suitable statistical method, for example as described in the Example herein.

In some embodiments, the disease is cancer.

According to a fourth aspect, the invention provides a method of identifying one or more biomarkers for the activity of a protein modifying enzyme comprising creating a dataset of modification sites according to the method of the first aspect of the invention or preparing a database according to the second aspect of the invention, wherein the modification sites in the single group are identified as the one or more biomarkers for the activity of a protein modifying enzyme.

According to a fifth aspect, the invention provides an *in vitro* method of determining that a test substance is a modulator of a protein modifying enzyme comprising treating a test sample with the test substance, identifying and/or quantifying modification sites on modified peptides in the test sample, creating a test dataset of modification sites from the modification sites identified and/or quantified in the test sample, further comprising:
(a) Comparing the test dataset with a dataset of modification sites created according to the method of the first aspect of the invention and/or
(b) Compiling said test dataset into a test database and comparing the test database with a database prepared according to the second aspect of the invention.

Preferred features for the second aspect and subsequent aspects of the invention are as for the first aspect *mutatis mutandis.*

The present invention will now be further described by way of reference to the following Examples which are present for the purposes of illustration only. In the Examples, reference is made to a number of Figures in which:

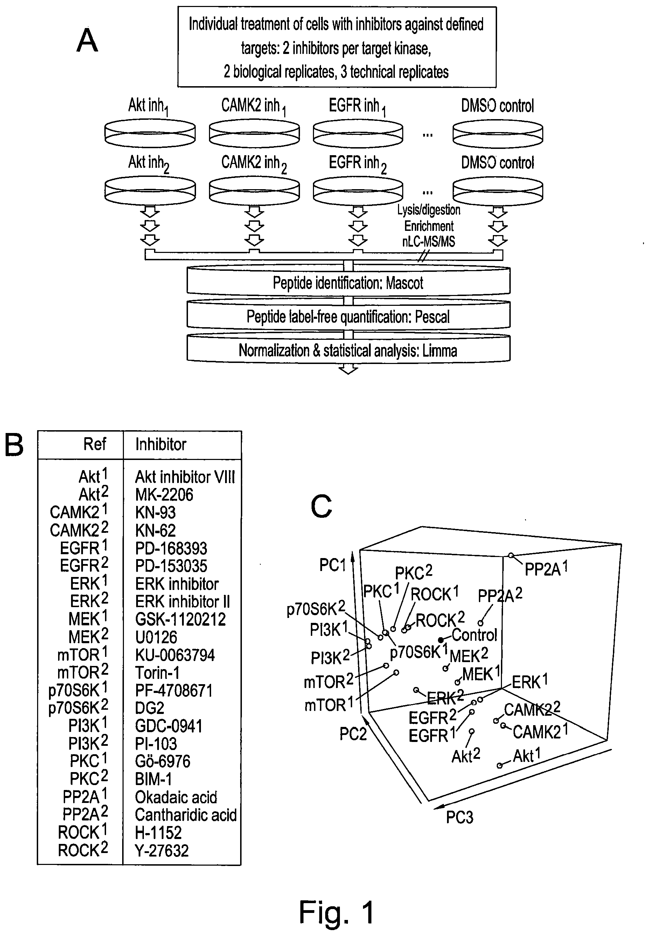

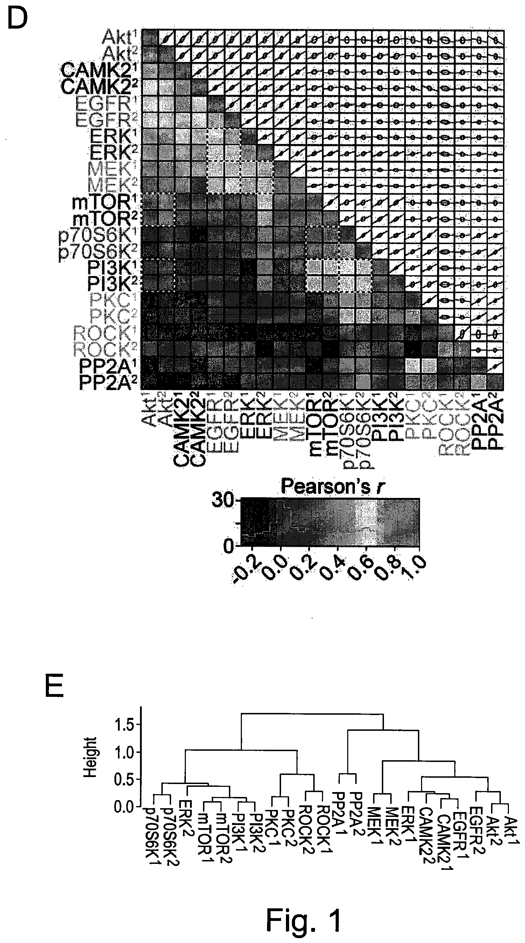

**Fig. 1****. Phosphoproteomics data elucidate the relationships between kinase inhibitors.** (A) Experimental design. Inh, inhibitor. (B) Kinase inhibitors used in the study. (C) Principal component analysis (PCA) of the 4,651 phosphorylation sites whose abundance was reduced significantly (adjusted P < 0.05) by at least one inhibitor treatment. Superscript numbers represent the inhibitor treatments as in (B). PC, principal component. (D) Lower triangle: Pearson correlation coefficients between each of the inhibitor treatments. Known kinase-kinase relationships are highlighted with white, dashed boxes. Upper triangle: pair-wise alignments of the 4,651 phosphorylation site log₂ fold-ratios for each inhibitor combination. The line in each box indicates the linear model formed between the two variables; the ellipse in each box represents one standard deviation from the mean in both dimensions. (E) Unsupervised, hierarchical clustering (Pearson correlation distance metric) of the mean log₂ ratios for peptides containing common phosphorylation motifs represented in the filtered 4,651 phosphorylation sites.

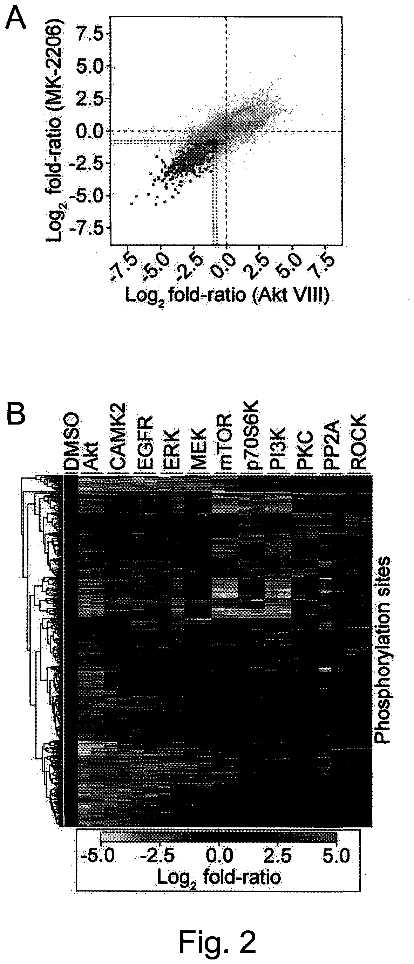

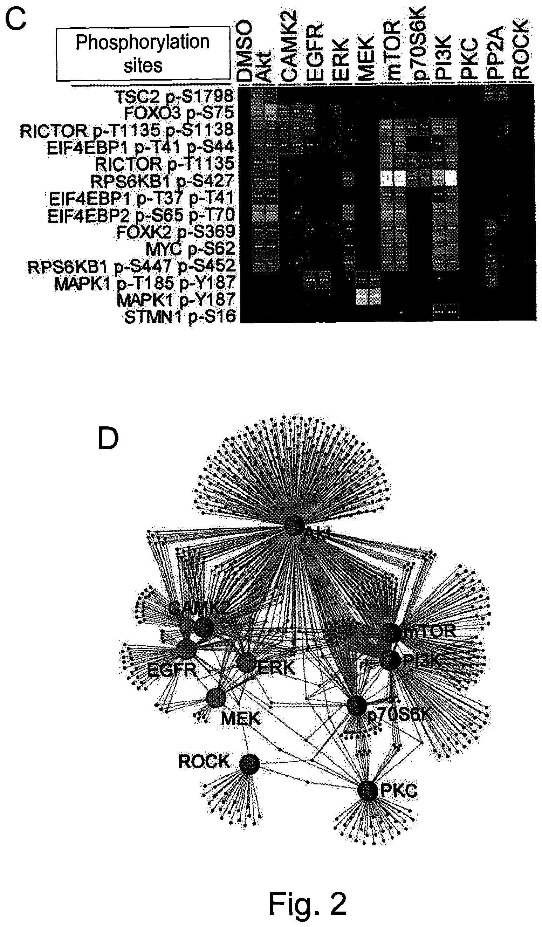

**Fig. 2****. Inference of a kinase signaling network topology from phosphoproteomics data.** (A) Phosphorylation site log₂ fold-ratios (versus DMSO control) for the two Akt inhibitors; MK-2206 and Akt inhibitor VIII. Dotted lines represent the thresholds for CTAM identification. The phosphorylation sites indicated using darker print in the negative quadrant of the graph (bottom-left) represent the compound-target activity markers (CTAMs) identified for Akt. Darkly shaded data points below the lower horizontal dotted line and left of the leftmost dotted line, FR ≤ -1.0, adj. P ≤ 0.1 for both inhibitors; darkly shaded data points below the upper horizontal dotted line and left of the rightmost dotted line, FR ≤ -0.75, adj. *P* ≤ 0.05 for both inhibitors; data from mixed thresholds between inhibitors are also shown. (B) 610 phosphorylation sites identified as being markers of compound-target activity for at least one kinase. (C) Illustrative examples of phosphorylation sites arranged into CTAM groups. * FR ≤ -0.75, adj. *P* ≤ 0.05; ** FR ≤ -1.0, adj. *P* ≤ 0.1; *** FR ≤ -1.0, adj. *P* ≤ 0.01. Shading as in (B). (D) An undirected, bipartite network graph showing all the identified activity markers. The layout of the network graph is based on a force-directed drawing algorithm. The large nodes represent the kinases targeted in the experiment (MAPK-associated (EGFR, MEK, ERK); PI3K/mTOR/p70S6K-associated (PI3K, mTOR, p70S6K); mixed association (Akt, CAMK2, ROCK, PKC)). Smaller nodes represent individual phosphorylation sites. Gray edges denote whether the phosphorylation site is a CTAM of the kinase to which it is connected.

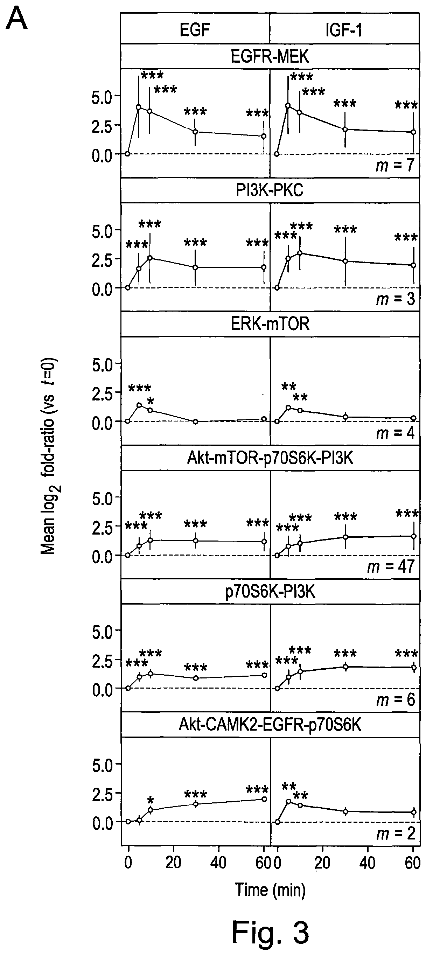

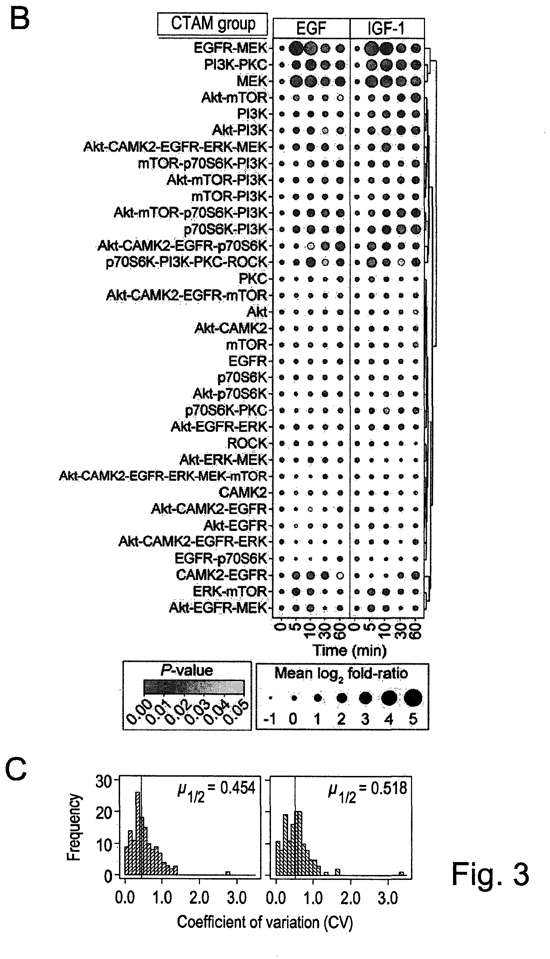

**Fig. 3****. Kinetics of CTAM group behavior upon growth factor stimulation.** (A) Temporal profiles of each of selected CTAM groups in cells treated with growth factors (EGF: left panels; IGF-1: right panels; m = number of phosphorylation sites quantified in the named CTAM group). Data points represent mean ± standard deviation (SD). *** *P* < 0.001; ** *P* < 0.01; * *P* < 0.05. (B) Temporal profiles for each of the CTAM groups represented in the dataset (with *m* ≥ 2). Data point sizes are proportional to the mean log₂ fold-ratio (versus t = 0 min) and shaded according to the statistical significance of enrichment. Unsupervised hierarchical clustering was based on the Euclidean distance metric. (C) Distribution of coefficient of variations for each of the quantified CTAM groups at each of the time-points. Vertical lines indicate the median.

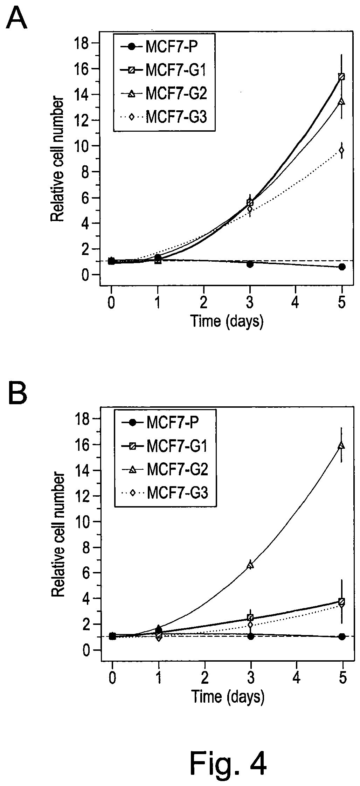

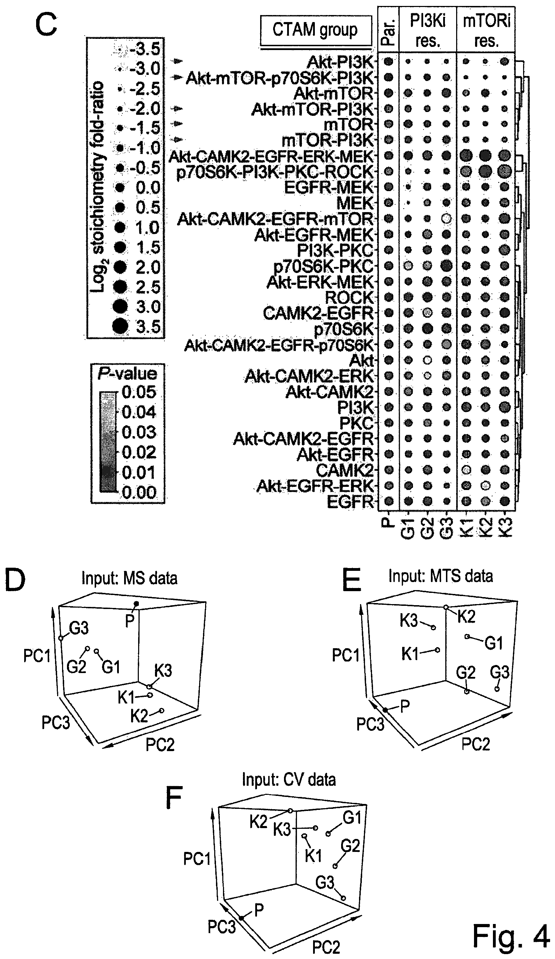

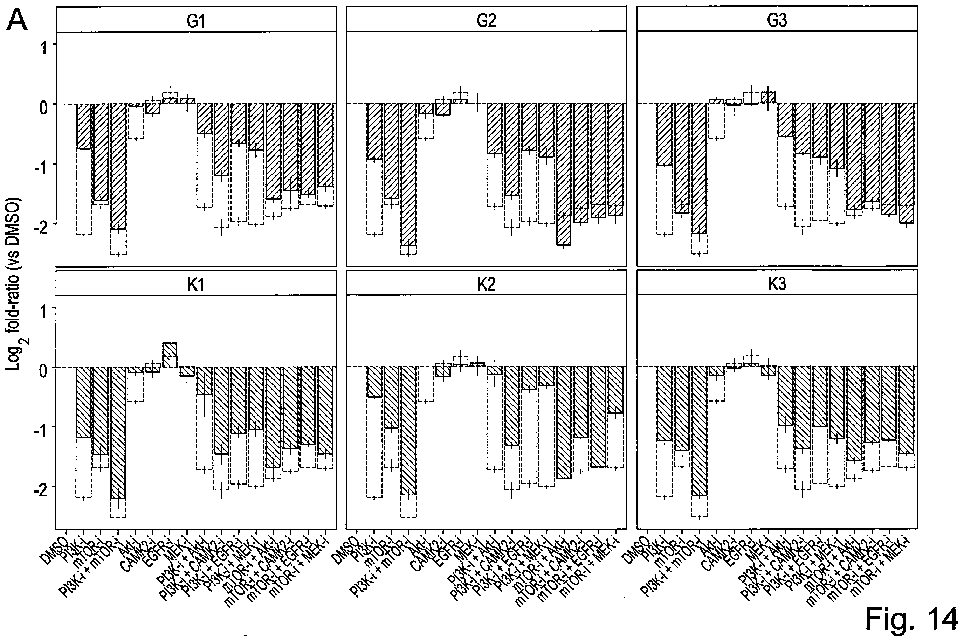

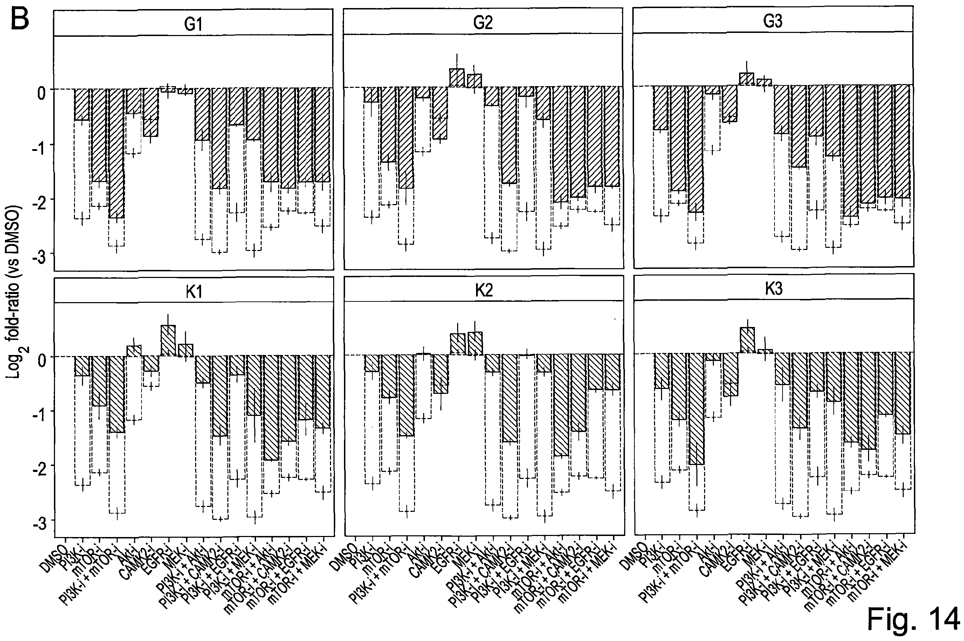

**Fig. 4****. Evolution of signaling network activity in cells resistant to mTORC1/2 and PI3K inhibitors.** Growth of parental and drug resistant MCF7 cell cultures in the presence of GDC-0941 (A) or KU-0063794 (B). Data points represent the mean ± SD. (C) CTAM group (with *m* ≥ 2) profiles for each of the resistant (res.) cell-lines compared to the parental (par.) cell-line. Dot sizes represent the mean log₂ fold-ratio of each CTAM group relative to parental cell-line, normalized to the unmodified protein abundance. Shading represents the significance of enrichment. Hierarchical clustering of the CTAM groups was based on the Euclidean distance metric. (D) PCA of the phosphoproteomic data shown in C. (E) PCA of cell viability data (measured by MTS) as a function of treatment with a panel of kinase inhibitors (shown in Detailed Appendix, Fig. 14A). (F) As in (E), however, cell viability was measured by crystal violet staining (shown in Detailed Appendix, Fig. 14B).

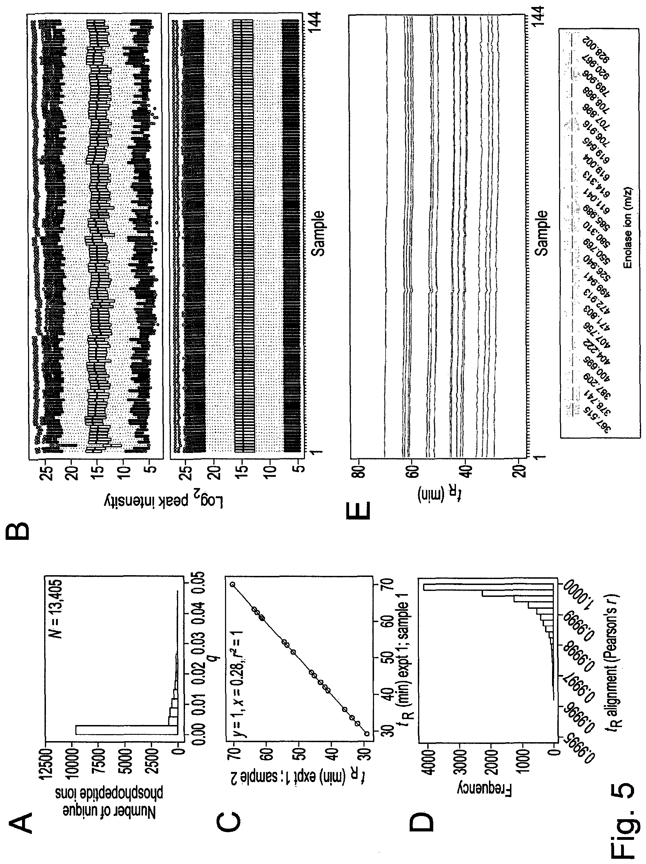

**Fig. 5****. Data summary for the kinase inhibitor-treated phosphoproteomes that define the network.** This is a quality control analysis of the data shown in Fig. 1 and 2. (A) Histogram showing the distribution of *q*-values within the phosphopeptide ion database (13,405 unique entries). (B) Box plots demonstrating the phosphopeptide ion log₂ intensity distributions for each sample pre-(top panel) and post-quantile normalization (bottom panel). (C) Illustrative example of an enolase peptide standard retention time (*t*_{R}) alignment between two samples in order to predict and account for retention time shifts. (D) Histogram showing the distribution of Pearson's correlation coefficients (*r*) for linear models formed between each pair-wise peptide standard alignment. (E) Line plot demonstrating the stability of enolase peptide standard retention times across the sample array. The shading of each line is specific to a single enolase peptide ion (as denoted in the key).

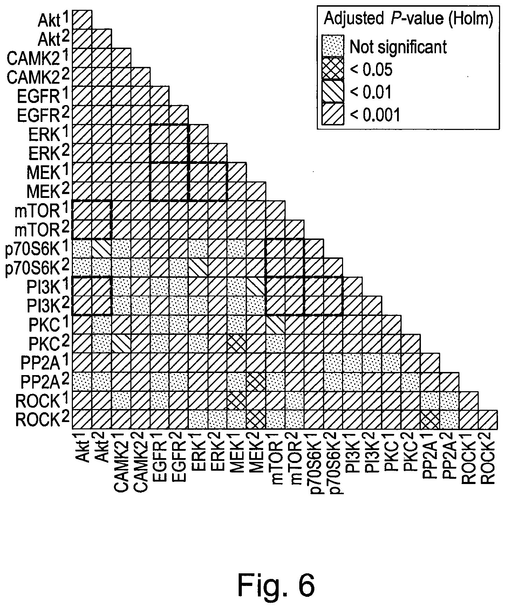

**Fig. 6****. Statistical significance of the correlations between inhibitor treatments.** Heat map demonstrating the significance of correlation between each inhibitor treatment (based on the 4,651 phosphopeptide ions found to be significantly down-regulated by at least one inhibitor pair, as in Fig. 1C). The significance is expressed as an *P*-value (calculated from Pearson's *r* and adjusted for multiple testing using the Holm procedure) and coded by shading accordingly. Known kinase-kinase relationships are highlighted with white, dashed boxes as in Fig. 1C.

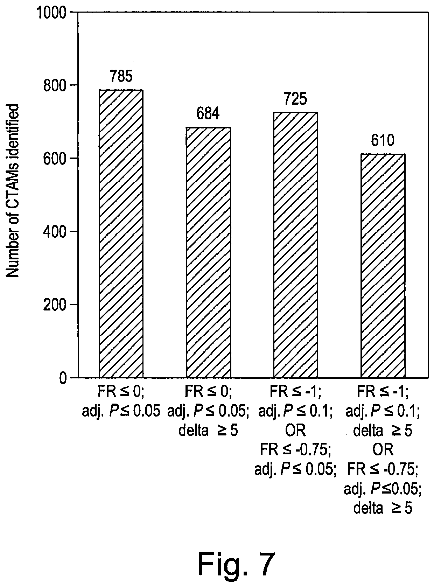

**Fig. 7****. Number of identified CTAMs as a function of selected statistical thresholds.** Bars represent the number of CTAMs identified under the indicated threshold conditions. FR = log₂ fold-ratio; adj. *P* = Benjamini-Hochberg adjusted *P*-value; delta = Mascot delta (δ) score.

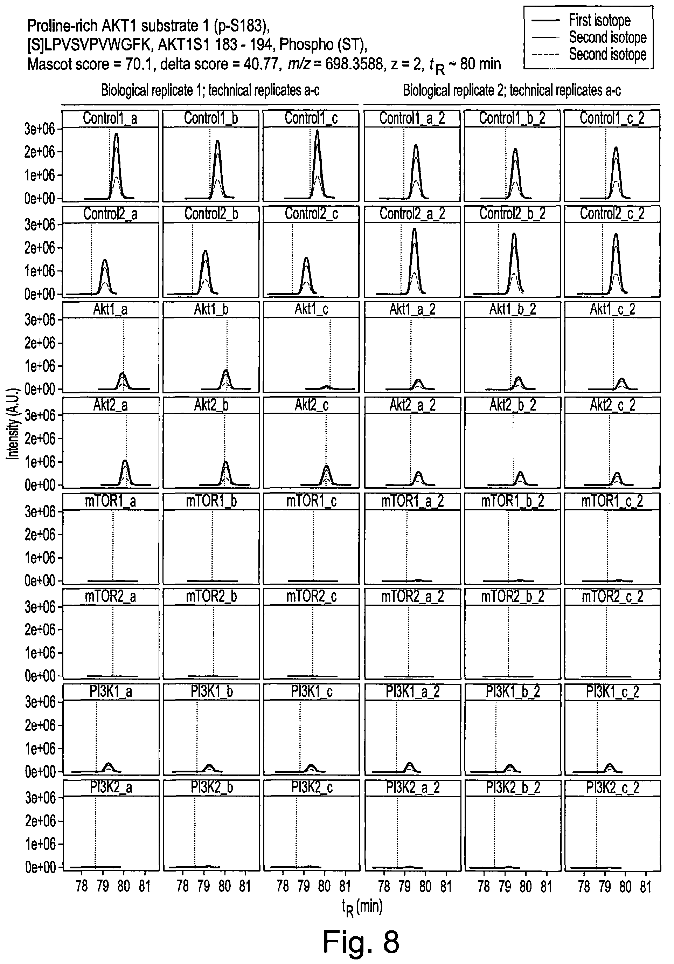

**Fig. 8****. Example of MS raw data.** Raw MS data supports the identification of AKT1S as a member of the Akt-mTOR-PI3K CTAM group. Raw MS data extracted ion chromatograms (XICs) for the phosphopeptide representing AKT1S (S183) at *m*/*z* 698.3588 (± 7 ppm). Lines represent the temporal elution profiles of the first, second, and third isotopes as outlined in the key (in each instance the highest peak represents the first isotope, the second highest peak represents the second isotope and the lowest peak represents the third isotope). Dotted lines represent the predicted retention time for the phosphopeptide in each sample. Akt1, Akt inhibitor VIII; Akt2, MK-2206; mTOR1, KU-0063794; mTOR2, Torin-1; PI3K1, GDC-0941; PI3K2, PI-103.

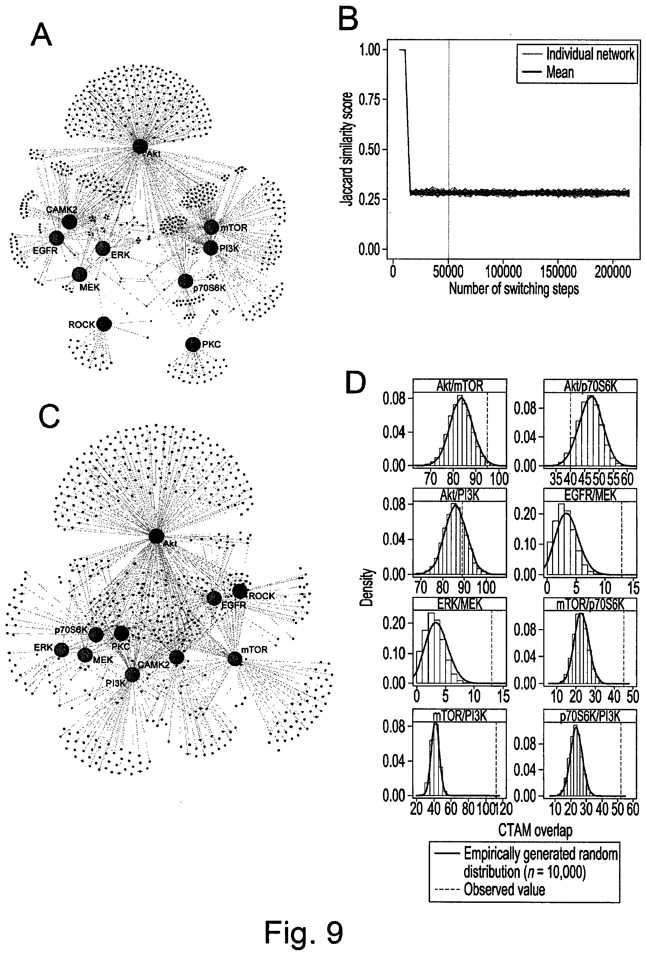

**Fig. 9****. Randomization of the defined network's topology.** (A) Empirically defined kinase signaling network as shown in Fig. 2D. (B) Optimization of the number of switching steps required to produce networks least similar to the original. Vertical line, the number of switching steps used in subsequent network randomization attempts. (C) An illustrative example of one of the 10,000 randomized networks (produced using 50,000 switching steps). (D) Distribution of CTAM overlaps between the indicated kinases in the randomized networks. Curved line, probability density function of the overlaps observed in the 10,000 randomized networks (as in C); vertical line, overlap observed in the real network (as in A).

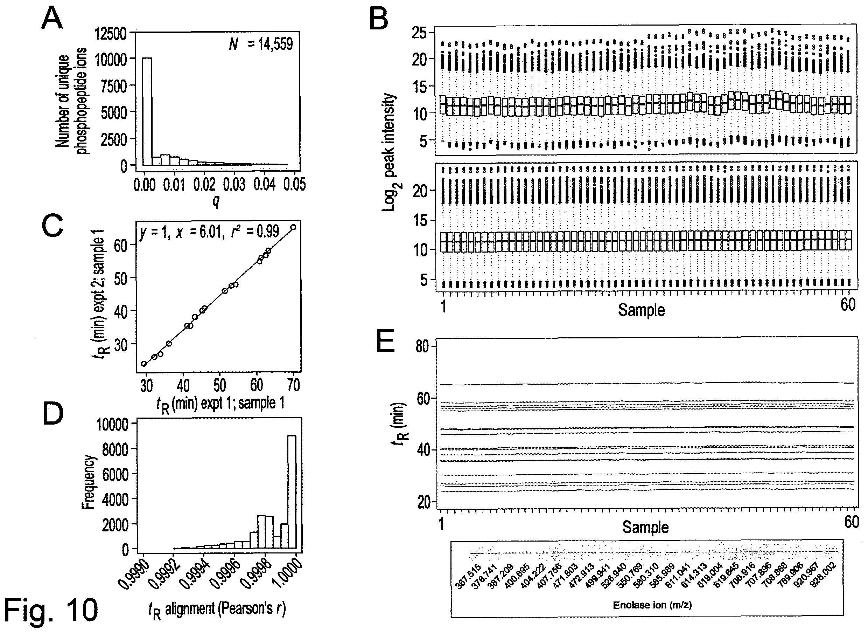

**Fig. 10****. Data summary for the stimulation of MCF7 cells with growth factors.** This is a quality control analysis of the data shown in Fig. 3. (A) Histogram showing the distribution of *q*-values within the phosphopeptide ion database (14,559 unique entries). (B) Box plots demonstrating the phosphopeptide ion log₂ intensity distributions for each sample pre- (top panel) and post-quantile normalization (bottom panel). (C) Illustrative example of an enolase peptide standard retention time (*t*_{R}) alignment between two samples, one from the first, network-defining experiment (Fig 5 - expt 1) and one from the growth factor stimulation experiment (expt 2), in order to predict and account for retention time shifts. (D) Histogram showing the distribution of Pearson's correlation coefficients (*r*) for linear models formed between each pair-wise peptide standard alignment in both the network-defining and growth factor experiments. (E) Stability of enolase peptide standard retention times across the sample array. The shading of each line is specific to a single enolase peptide ion (as denoted in the key).

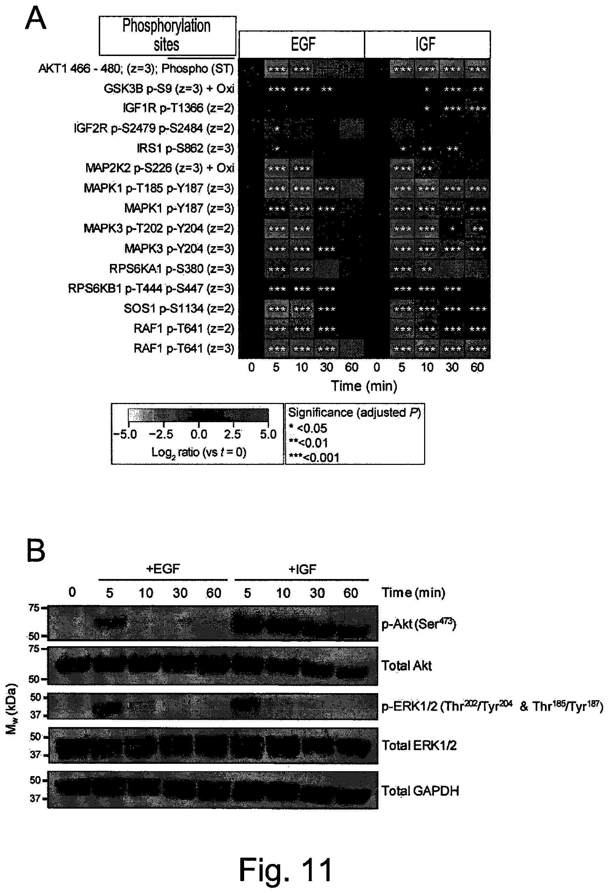

**Fig. 11****. Illustrative examples of the dynamics of phosphorylation sites downstream of EGFR and IGF-1R.** Heat map showing the log₂ fold-ratios (vs *t* = 0) for several phosphorylation sites known to be downstream of EGFR and IGF-1R following stimulation with either of the growth factors. Phosphorylation sites possessing a Mascot delta score ≥ 10 are presented as the protein name, peptide charge (*z*) ± any other peptide modifications. Those possessing a Mascot delta score ≤ 10 are represented as the protein name, residues between which the phosphorylation site resides, peptide charge, and the predicted modification. Asterisks indicate statistical significance (adjusted P-value) as outlined in the legend. (B) Western blots for known EGF/IGF-sensitive phosphorylation sites activating the Akt-mTOR-PI3K and MAPK pathways.

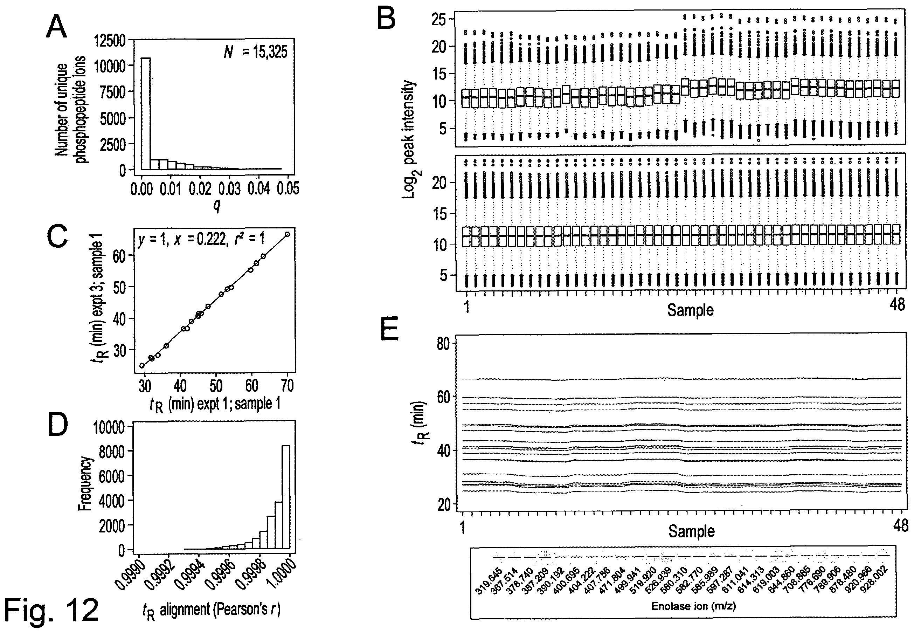

**Fig. 12****. Data summary for the quantitative phosphoproteomics and proteomics comparison of resistant cell-lines and parental cells.** This is a quality control analysis of the data shown in Fig. 4. (A) Histogram showing the distribution of *q*-values within the phosphopeptide ion database (15,325 unique entries). (B) Box plots demonstrating the phosphopeptide ion log₂ intensity distributions for each sample pre- (top panel) and post-quantile normalization (bottom panel). (C) Illustrative example of an enolase peptide standard retention time (*t*_{R}) alignment between two samples, one from the first, network-defining experiment (Fig 5 - expt 1) and one from the resistant cell-line comparison experiment (expt 3), in order to predict and account for retention time shifts. (D) Histogram showing the distribution of Pearson's correlation coefficients (*r*) for linear models formed between each pair-wise peptide standard alignment in both the network-defining and resistant cell-line experiments. (E) Stability of enolase peptide standard retention times across the sample array. The shading of each line is specific to a single enolase peptide ion (as denoted in the key).

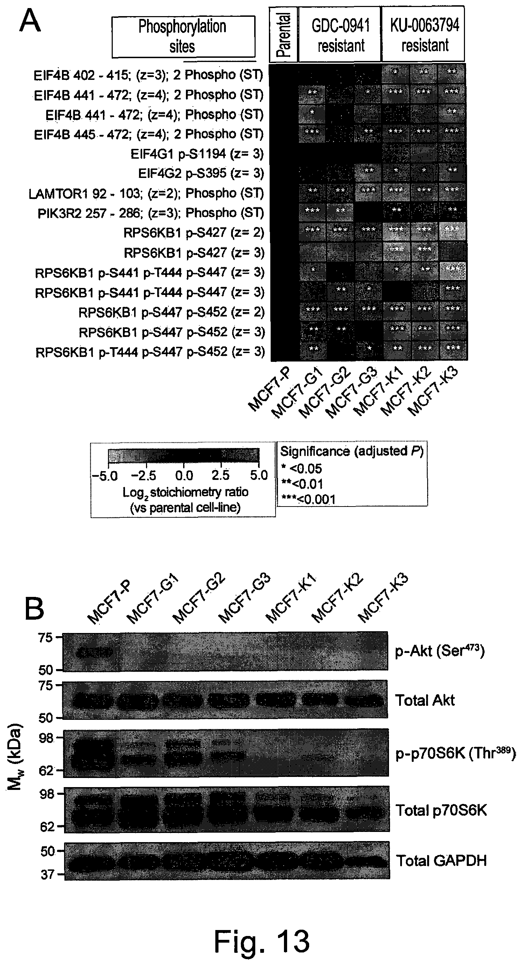

**Fig. 13****. Illustrative examples of phosphorylation sites reported to be downstream of PI3K and mTORC1/2 in resistant cell-lines treated with inhibitors.** Heat map demonstrating the log₂ stoichiometry ratios (log₂ fold-ratio of the phosphorylation site/log₂ fold-ratio of the protein) vs the parental cell-line of several phosphorylation sites known to be downstream of PI3K and mTORC1/2. Phosphorylation sites possessing a Mascot delta score ≥ 10 are presented as the protein name, peptide charge (*z*) ± any other peptide modifications. Those possessing a Mascot delta score ≤ 10 are represented as the protein name, residues between which the phosphorylation site resides, peptide charge, and the predicted modification. Asterisks indicate statistical significance (adjusted P-value) as outlined in the legend. (B) Western blots for known markers of Akt-mTOR-PI3K pathway activity.
**Fig. 14****. Cell viability data for the resistant and parental cell-lines treated with a panel of kinase inhibitors.** Bars represent mean log₂ fold-ratio of cell viability (vs DMSO) for each kinase inhibitor alone or in combination in each of the resistant cell-lines (MCF7-G, solid bars, dark shading, upper panels (G1, G2, G3); MCF7-K, solid bars, light shading, lower panels (K1, K2, K3); MCF7-P, dotted white bars (G1, G2, G3, K1, K2, K3)) as determined by the MTS assay (A) and crystal violet assays (B). Error bars represent standard deviation from the mean in each case (n = 3). These data were the input for the PCA presented in Fig. 4E. PI3K-i = 1 µM GDC-0941; mTOR-i = 1 µM KU-0063794; Akt-i = 1 µM MK-2206; CAMK2=i = 5 µM KN-93; EGFR-i = 1 µM PD-153035; MEK-i = 0.5 µM GSK-1120212.

### EXAMPLES

### Methods

**Cell lines.** The parental MCF7 cell line and MCF7-G1, MCF7-G2, MCF7-G3, MCF7-K1, MCF7-K2 and MCF7-K3 resistant cells were cultured in DMEM (supplemented with 10% fetal-bovine serum and 100 U.mL⁻¹ penicillin/streptomycin) at 37°C in a humidified atmosphere at 5% CO₂. After treatment, as indicated in the text, cells were lysed in urea lysis buffer and proteins digested with trypsin.

**Mass spectrometry-based phosphoproteomics.** Phosphorylated peptides were enriched using TiO₂ beads (GL Sciences) similarly to that previously described (Casado et al. (2013) Sci. Signal. 6(268):rs6., Montoya et al (2011) Methods 54(4):370-378) with some modifications, and analyzed by LC-MS/MS using a LTQ-Orbitrap mass spectrometer. Peptides were identified by means of Mascot searches against the SwissProt human protein database. Peptide quantification was achieved using Pescal as previously described (Casado et al. (2013) Sci. Signal. 6(268):rs6.).

**Statistical analysis.** Following quantile normalization of the data (Bolstad et al (2003) Bioinformatics 19(2):185-193), the magnitude and statistical significance of differences between conditions were computed using an empirical Bayes shrinkage of standard deviations (Smyth (2004) Stat. Appl. Genet. Molec. Biol. 3(1):Article3) using the *limma* package within the R computing envrionment (Smyth (2005) Limma: linear models for microarray data (Springer New York); R Core Team (2013) R: A language and environment for statistical computing (R Foundation for Statistical Computing)). The abundance of CTAMs was monitored systematically using KSEA (kinase-substrate enrichment analysis), as described previously (Casado et al. (2013) Sci. Signal. 6(268):rs6; Casado et al. (2013) Genome Biol. 14(4):R37; Montoya et al (2011) Methods 54(4):370-378; Alcolea et al (2012) Mol. Cell. Proteomics 11(8):453-466).

A more detailed description of these methods is provided in the Detailed Materials and Methods, below.

### Detailed Materials and Methods

**Reagents.** Akt inhibitor VIII, KU-0063794, GDC-0941, PI-103 and Y-27632 were obtained from Chemdea. KN-93, KN-62, PD-168393, PD-153035, ERK inhibitor, ERK inhibitor II, U0126, PF-4708671, DG2, Gö-6976, bisindolylmaleimide-1, okadaic acid, cantharidic acid, and H-1152 were obtained from Calbiochem (Merck). GSK-1120212 and MK-2206 were obtained from Selleckchem. Torin-1 was obtained from Axon Medchem. Human recombinant EGF (AF-100-15) and IGF-1 (100-11) were purchased from Peprotech.

**Cell-lines.** MCF7 cells (identity confirmed by genotyping analysis) were routinely maintained in DMEM (supplemented with 10% fetal-bovine serum and 100 U.mL⁻¹ penicillin/streptomycin) at 37°C in a humidified atmosphere at 5% CO₂. Resistant MCF7 cell-lines, derived from the original parental cells, were obtained by gradually increasing the concentrations of GDC-0941 or KU-0063794 concentrations (starting at 100 nM up to a maximum of 1 µM) over a period of approximately six months. The resultant clones (MCF7-G1:G3 and MCF7-K1:K3) were then routinely maintained as above in the presence of either 1 µM GDC-0941 or KU-0063794 (in DMSO) for MCF7-G and MCF7-K cells, respectively.

**Cell lysis and protein digestion.** Cells were split and seeded at 4 x 10⁵ cells.plate⁻¹, 72 hours prior to the experiment. The medium in each plate was replaced 24 hours prior to the experiment. Each experiment was performed in biological duplicate. For the network definition experiments, cells were treated with each of the inhibitors or vehicle (DMSO) for 1 hour (Table 1). For the growth factor stimulation experiments, cells were starved for 24h and were subsequently treated with either 50 ng.mL⁻¹ EGF or IGF for 0, 5, 10, 30, or 60 minutes. For the resistant cell-line experiments, no additional treatments were performed. For the rewiring experiments in the MCF7-P and -G1:3 cell-lines, cells were starved for 24h and subsequently treated with 50 ng.mL⁻¹ EGF for 0, 30, 60, or 120 minutes. Following the treatments, cells were washed three times with ice-cold phosphate-buffered saline supplemented with 1 mM Na₃VO₄ and 1 mM NaF, and lysed with urea lysis buffer [8M urea in 20 mM HEPES (pH 8.0), supplemented with 1 mM Na₃VO₄, 1 mM NaF, 1 mM Na₂P₂H₂O₇, and 1 mM β-glycerol phosphate]. Lysates were further homogenized by probe sonication (three 10s pulses) and insoluble material removed by centrifugation. Protein concentration was estimated using the Bradford or Smith assay. After normalizing each condition to a common protein concentration (250 or 500 µg), each sample was reduced and alkylated by sequential incubation with 10 mM dithiothreitol and 16.6 mM iodoacetamide for 30 min at room temperature, in the dark. For protein digestion, the urea concentration was reduced to 2M by the addition of 20 mM HEPES (pH 8.0). Immobilised tosyl-lysine chloromethyl ketone (TLCK)-trypsin [20 p-toluenesulfonyl-L-arginine methyl ester (TAME) units.mg⁻¹] was then added, and samples incubated overnight at 37°C. Trypsin beads were removed by centrifugation and the resultant peptide solutions desalted with 30 mg Oasis-HLB cartridges (Waters, Manchester, UK), using a vacuum manifold. Briefly, Oasis cartridges were conditioned with acetonitrile (ACN) and equilibrated with wash solution (0.1% TFA, 1% ACN). Peptides were loaded into the cartridges and washed with 1 mL wash solution. Finally, peptides were eluted with glycolic acid buffer 1 (1M glycolic acid, 5% TFA, 50% ACN). When analyzing total protein, aliquots of the parental and original resistant cell-line lysates were taken and processed as above with the exception that the samples were eluted from the OASIS cartridges with 60% ACN (40% H₂O), dried-down using a SpeedVac, and stored at -80°C until further analysis.

**Phosphopeptide enrichment.** Phosphorylated peptides were enriched using TiO₂ (GL Sciences) similarly to that previously described (1, 2) with some modifications outlined in Detailed Methods. Briefly, peptide eluents were normalized with glycolic acid buffer 2 (1M glycolic acid, 5% TFA, 80% ACN) and incubated with 50 µL of TiO₂ (a 50% slurry in 1% TFA), for 5 min at room temperature.

Beads were pelleted by centrifugation, 80% of the supernatant removed and stored on ice. The remaining solution was used to re-suspend the beads, and the beads packed into pre-washed, empty, PE-filtered spin-tips (Glygen, MD, USA) by centrifugation. Residual beads were re-suspended with a further glycolic acid buffer 2 and packed into the tips. The remaining peptide solution was removed from ice and washed over the packed TiO₂ beads by centrifugation. The packed tips were then sequentially washed with glycolic acid buffer 2, ammonium acetate buffer (100 mM ammonium acetate; 25% ACN), and 10% ACN, the latter being repeated in triplicate. Phosphopeptides were eluted by centrifugation with four sequential washes of 5% NH₄OH in 10% ACN. The resulting phosphopeptide solutions were snap-frozen, dried in a SpeedVac, and stored at -80°C.

**LC-MS/MS phosphoproteomics analysis.** Dried phosphopeptide extracts were re-suspended in 20 µL of 0.1% TFA (5% ACN) containing 20 fmol.µL⁻¹ enolase digest (Waters, Manchester, UK). For each technical replicate, 3.0 µL of each sample was loaded into a Dionex Ultimate nRSLC 3000 LC system (Thermo Fisher Scientific) coupled online to an LTQ-Orbitrap-Velos mass spectrometer (Thermo Fisher Scientific). The samples were separated on an 85 min linear gradient between 5 and 35% ACN on a Acclaim PepMap RSLC column (25 cm x 75 µm, 2 µm, 100 Å), and the top seven most intense multiply charged ions in each MS¹ scan were selected for collision-induced dissociation fragmentation (with multistage activation enabled). The resolution of the MS¹ was set to 30,000 FWHM. Each sample was run in triplicate.

**LC-MS/MS proteomics analysis.** Dried peptide extracts were re-suspended to a final concentration of 0.5 µg.µL⁻¹ in 0.1% TFA, 3% ACN. For each technical replicate, 4.0 µL of each sample was loaded into the LC-MS/MS system described above. The samples were separated on a 120 minute linear gradient between 3 and 32% ACN, and the top ten most intense multiply charged ions in each MS¹ scan were selected for collision-induced dissociation fragmentation. The resolution of the MS¹ was set to 30,000 FWHM. Each sample was run in duplicate.

**Phosphopeptide identification and quantification.** Peptide identification was performed by matching deisotoped, MS/MS data to the Uniprot-Swissprot human protein databases (October 2012 release, containing 20,233 entries), utilizing the Mascot server version 2.3. Mascot Distiller version 2 was used to generate peak lists in the mascot generic format. The samples were also searched against a scrambled version of the former database. Mass tolerances were set to 10 ppm and 600 mmu for the precursor and fragment ions respectively. For the phosphoproteomics experiments, allowed variable modifications were phospho-Ser, phospho-Thr, phospho-Tyr, pyro-Glu (N-terminal), and oxidation-Met. The identified phosphopeptides from each of the samples were collated, curated, and a false-discovery rate calculated (by comparison to the scrambled database) using in-house scripts. Unique phosphopeptides ions with FDR < 5% were then included in the subsequent analyses. Using these settings >95% of peptides had a probability of FDR <1% (Fig. 5). The databases from the growth factor and resistant cell-line experiments were merged with the original network discovery database to ensure addition of the identified activity markers. Peptide quantification was performed as described before by our group (1-4) and others (5, 6). Briefly, Pescal software (written in Python v2.7) was used to obtain peak heights of extracted ion chromatograms of each of the phosphopeptide ions in the database, across all of the samples being compared. The retention times of each phosphopeptide ion in each sample were predicted by aligning the enolase standard peptides spiked into each sample, using an in-house linear modeling algorithm, and the subsequent integration of chromatographic peaks obtained from extracted ion chromatograms for each phosphopeptide in each sample. This approach is similar to that reported for the alignment of retention times in multiplexed selected reaction monitoring experiments (7). The mass-to-charge (*m*/*z*) and retention time (*t*_{R}) tolerances were set to 7 ppm and 1.5 min, respectively. Proteomics data were processed as above; however, protein identity was inferred only for those possessing ≥ 2 peptides with Mascot peptide scores ≥ 20 and a Mascot protein score ≥ 60.

**Statistical analysis and data visualization.** Following quantile normalization of the data (8), robust statistical analysis to assess the magnitude and significance of phosphorylation changes was performed using a linear modeling strategy and empirical Bayes shrinkage of standard deviations (9). The resulting *P*-values were then corrected for multiple testing using the Benjamini-Hochberg procedure. All of the described analysis was performed using the *limma* package (v3.16.2) within the R statistical computing environment (v3.0.0) (10, 11). The datasets were visualized using a combination of individual R packages, namely: *ggplot2, gplots, reshape2, igraph,* and *RCytoscape,* (12-16). Network graphs were constructed within the Cytoscape software package (v2.8.3) (17) and randomized using the *BiRewire* R package (18). Log₂ stoichiometry fold-ratios were calculated by subtracting the calculated log₂ ratio (vs control) of the total protein from the log₂ fold-ratio (vs control) of the individual phosphorylation site.

**Kinase-substrate enrichment analysis and motif analysis.** The abundance of each of the identified markers was monitored systematically using KSEA (kinase-substrate enrichment analysis), as described previously (1). Briefly, the phosphopeptide ions identified within the growth factor and resistant cell-line experiments that were previously identified as being CTAMs were systematically extracted from the dataset. The mean of the log₂ fold-ratios (versus control) of the phosphorylation sites representing each CTAM group were then calculated alongside the standard deviation (*δ*) for each mean. The significance of enrichment was then determined using a Z-score based approach and expressed as *P*-values, using the following formula: Z = (*Sₘ* - *µₜ*).*m*^{1/2}/*δₜ*; where *Sₘ* = mean of the CTAM group log₂ fold-ratios; *µₜ* = mean of the total dataset log₂ ratios; *m* = number of individual phosphopeptides within the CTAM group; and *δₜ* = standard deviation of the total dataset log₂ ratios (vs control) (19). The motif analyses were performed as in (1). Briefly, the phosphopeptides under investigation were first grouped according to which consensus/common motif they represent. The mean log₂ fold-ratio (vs DMSO control) was then calculated for each motif group under each condition.

**CTAM identification algorithm.** Phosphorylation sites were first filtered to only include those possessing a Mascot δ-score ≥ 5. Activity markers for each kinase inhibitor were then identified by selecting phosphorylation sites with a log₂ fold-ratio ≤ 1 (i.e. a 50% linear reduction in abundance) and adjusted *P* ≤ 0.1, or with those with a smaller change (log₂ fold-ratio ≤ 0.75), but greater significance (adjusted *P* ≤ 0.05) for each inhibitor. Phosphorylation sites that met the above thresholds under both inhibitor treatments targeting the same kinase were subsequently identified as compound-target activity markers (CTAMs).

**Cell viability assays.** Parental MCF7, G1:3 and K1:3 cell-lines were seeded in 96-well plates at a density of approximately 5,000 cells.well⁻¹, in biological triplicate. After 24 hours, the cells were then treated with the indicated concentrations/combinations of GDC-0941, KU-0063794, MK-2206, KN-93, PD-153035, GSK-1120212 or DMSO (as Table 1). Following a 48 hour treatment with the inhibitors, cell proliferation was determined with the MTS assay (Promega, WI, USA) or crystal violet stain. For the MTS assays the absorbance (490 nm) of each well was determined using a spectrophotometer following a 120 min incubation with the reagent, this measurement being acquired in duplicate. For the crystal violet staining, cells were fixed with 100 µL.well⁻¹ paraformaldehyde (4% w/v in PBS) for 30 min on ice, stained with 100 µL.well⁻¹ crystal violet stain (0.5% w/v in 20% MeOH) for 10 min at room temperature and washed twice with ddH₂O. Following the washes, the crystal violet was re-suspended using 100 µL.well⁻¹ Sorensen's buffer (0.1M sodium citrate, 50% EtOH, pH 4.2) and the absorbance (540 nm) of each well measured in duplicate. The ratio of the absorbance of each concentration data-point versus the absorbance of control (DMSO) treated cells was then calculated, log-transformed, and the mean of the each set of replicates calculated.

**Table 1. Small-molecule inhibitors used in the experiments**

| **Inhibitor** | **Concentration used (µM)** | **Primary target** | **Off-targets from MRC database (>50% inhibition at given concentration)** | **ChEMBL ID** | **Off-targets from ChEMBL** | **Reference material** |
|---|---|---|---|---|---|---|
| Akt inhibitor VIII | 1 | Akt1/2/3 | CAMK1 (1 µM) | 258844 | - | (20) |
| MK-2206 | 1 | Akt1/2/3 | - | 1079175 | - | (21, 22) |
| KN-62 | 5 | CAMK2α/ β/γ/δ | PRAK, CAMK1, DYRK1A, Lck (10 µM) | 155333 | P2RX7 | (23) |
| KN-93 | 10 | CAMK2α/ β/γ/δ | TrkA, smMLCK (10 µM) | 28234 | - | (24) |
| PD-168393 | 1 | EGFR | - | 285063 | ERBB2/4, BMX, BTK, BLK, JAK3 | (25) |
| PD-153035 | 10 | EGFR | - | 29197 | ABL1, ERBB2, MKNK1, F16P1 | (26) |
| ERK inhibitor I | 40 | ERK1/2 | - | 1403932 | - | (27) |
| ERK inhibitor II (FR18020 4) | 30 | ERK1/2 | - | 259551 | MAPK14 | (28) |
| GSK-1120212 | 0.5 | MEK1/2 | - | - | - | (29, 30) |
| U0126 | 10 | MEK1/2 | MKK1 (10 µM) | 100473 | - | (30, 31) |
| KU-0063794 | 1 | mTORC1 /2 | - | 1078983 | - | (32) |
| Torin-1 | 1 | mTORC 1 /2 | - | 1256459 | PI3Kα/δ/γ, p85α, PI3K-C2α/β, Vps34, DNA-PK | (33) |
| PF-4708671 | 20 | p70S6K (S6K1) | MSK1, RSK1 (1 µM) | - | S6Kα5 | (34) |
| DG2 | 5 | p70S6K (S6K1) | - | 1254209 | - | (35) |
| GDC-0941 | 1 | PI3K (class I) | CLK2 (1 µM) | 573393 | Akt1, mTOR, HIPK2/3, MYLK4, CLK2, MAPK10, FLT3, JAK1, RIOK2 | (36, 37) |
| PI-103 | 1 | PI3K (class I) | - | 521851 | mTOR, DNA-PK, Akt1 | (38) |
| Gö-6976 | 1 | PKCα/β/δ /γ | MKK1, BRSK2, ERK2/8, CAMKKα, MNK2, DYRK1A/3, SmMLCK, HIPK2, Aurora C, GSK3β, Aurora B, PIM1, MELK, AMPK, MARK3, CAMKKβ, S6K1, PDK1, CDK2, RSK1, PAK4/5/6, MSK1, PKD1, PIM3, RSK2, PRK2, PHK, CHK1, MST2 (0.1 µM) | 302449 | PKD1 | (39) |
| Bisindolyl maleimide -I | 1 | PKCα/θ/η /γ/ε | MKK1, ROCK2, Lck, PKA, Akt1, MAPKAP-K2, CHK1, PKD1, AMPK, S6K1, GSK3β, CDK2, SGK1, DYRK1A, MSK1, PHK, RSK1 (10 µM) | 7463 | CDK4/CCN D1, GSK3β, PIM1 | (40) |
| Okadaic acid | 1 | PP2A | - | 280487 | - | (41) |
| Cantharidi c acid | 10 | PP2A | - | 275516 | - | (42) |
| H-1152 | 5 | ROCK | MNK1, MARK3, MELK, EPH-A2, RSK2, MSK1, BRSK2, FGF-R1, PKA, AMPK, RSK1, PHK, Aurora B/C, PRK2 (1 µM) | 406821 | PKA | (43) |
| Y-27632 | 10 | ROCK | PHK, MST2, RSK2, MSK1, MNK1, RSK1, AMPK, PRK2 (1 µM) | - | PKCε | (44) |

**Cell proliferation assays.** Parental MCF7, G1:3 and K1:3 cell-lines were seeded in 12-well plates at a density of approximately 80,000 cells.well⁻¹, in biological triplicate. After the indicated time period, cells were washed with PBS, trypsinized and counted using a Beckman Coulter Vi-CELL XR cell counter. Second-order polynomials where fitted to the data using the *ggplot2* package (v0.9.3.1) within R (v3.0.0).

**Immunoblotting.** Cells were lysed in lysis buffer (50 mM Tris-HCI [pH 7.4], 1 mM EDTA, 150 mM NaCl, 1% Triton-X100; supplemented with 1 mM Na₃O₄, 1 mM NaF, 1 mM β-glycerol phosphate, 2.5 mM Na₂P₂H₂O₇, 1 mM PMSF, and 1X protease inhibitor cocktail [Sigma Aldrich]). Samples were resolved by SDS-polyacrylamide gel electrophoresis using either 12% or 4-15% gradient precast gels (BioRad). Proteins were transferred to PVDF membranes using the BioRad Trans-Blot Turbo system as per manufacturer's instructions. Once transferred, membranes were blocked with 5% w/v skimmed milk powder in Tris-buffered saline supplemented with 0.1% Tween-20. Blocked membranes were incubated with primary and secondary antibodies and developed with SuperSignal West Pico Chemiluminescent Substrate (Thermo Scientific). Primary antibodies were used at 1:500 or 1:1000 dilution and secondary antibodies were used at 1:5000 dilution.

### Supplementary References (these apply only to the Detailed Materials & Methods, above)

1. Casado P, et al. (2013) Kinase-substrate enrichment analysis provides insights into the heterogeneity of signaling pathway activation in leukemia cells. Sci. Signal. 6(268):rs6.
2. Montoya A, Beltran L, Casado P, Rodriguez-Prados JC, & Cutillas PR (2011) Characterization of a TiO(2) enrichment method for label-free quantitative phosphoproteomics. Methods 54(4):370-378.
3. Casado P & Cutillas PR (2011) A self-validating quantitative mass spectrometry method for assessing the accuracy of high-content phosphoproteomic experiments. Mol. Cell. Proteomics 10(1):M110 003079.
4. Cutillas PR & Vanhaesebroeck B (2007) Quantitative profile of five murine core proteomes using label-free functional proteomics. Mol. Cell. Proteomics 6(9):1560-1573.
5. Tsou CC, et al. (2010) IDEAL-Q, an automated tool for label-free quantitation analysis using an efficient peptide alignment approach and spectral data validation. Mol. Cell. Proteomics 9(1):131-144.
6. Mann B, et al. (2008) ProteinQuant Suite: a bundle of automated software tools for label-free quantitative proteomics. Rapid Commun. Mass Spectrom. 22(23):3823-3834.
7. Escher C, et al. (2012) Using iRT, a normalized retention time for more targeted measurement of peptides. Proteomics 12(8):1111-1121.
8. Bolstad BM, Irizarry RA, Astrand M, & Speed TP (2003) A comparison of normalization methods for high density oligonucleotide array data based on variance and bias. Bioinformatics 19(2):185-193.
9. Smyth GK (2004) Linear models and empirical bayes methods for assessing differential expression in microarray experiments. Stat. Appl. Genet. Molec. Biol. 3(1):Article3.
10. Smyth GK (2005) Limma: linear models for microarray data (Springer New York).
11. R Core Team (2013) R: A language and environment for statistical computing (R Foundation for Statistical Computing).
12. Wickham H (2009) ggplot2: elegant graphics for data analysis (Springer New York).
13. Warnes GR et al. (2013) gplots: Various R programming tools for plotting data*.*
14. Wickham H (2007) Reshaping data with the reshape package. J. Stat. Softw. 21(12):1-20.
15. Csardi G & Nepusz T (2006) The igraph software package for complex network research. InterJournal Complex Systems: 1695.
16. Shannon P, et al. (2013) RCytoscape: tools for exploratory network analysis. BMC Bioinformatics 14:217.
17. Shannon P, et al. (2003) Cytoscape: a software environment for integrated models of biomolecular interaction networks. Genome Res. 13(11):2498-2504.
18. Gobbi A, et al. (2014) Fast randomization of large genomic datasets while preserving alteration counts. Bioinformatics 30(17):i617-623.
19. Kim SY & Volsky DJ (2005) PAGE: parametric analysis of gene set enrichment. BMC Bioinformatics 6:144.
20. Lindsley CW, et al. (2005) Allosteric Akt (PKB) inhibitors: discovery and SAR of isozyme selective inhibitors. Bioorg. Med. Chem. Lett. 15(3):761-764.
21. Rehan M, Beg MA, Parveen S, Damanhouri GA, & Zaher GF (2014) Computational insights into the inhibitory mechanism of human AKT1 by an orally active inhibitor, MK-2206. PLoS ONE 9(10):e109705.
22. Hirai H, et al. (2010) MK-2206, an allosteric Akt inhibitor, enhances the antitumor efficacy by standard chemotherapeutic agents or molecular targeted drugs in vitro and in vivo. Mol. Cancer Ther. 9(7):1956-1967.
23. Sumi M., et al. (1991) The newly synthesized selective Ca2+/calmodulin dependent protein kinase-II inhibitor KN-93 reduces dopamine contents in PC12H cells. Biochem. Biophys. Res. Commun. 181(3):968-975.
24. Tokumitsu H, et al. (1990) KN-62, 1-N,O-bis(5-isoquinolinesulfonyl)-N-methyl-L-tyrosyl-4-phenylpiperazin E, a specific inhibitor of Ca2+/calmodulin-dependent protein kinase-II. J. Biol. Chem. 265(8):4315-4320.
25. Fry DW, et al. (1998) Specific, irreversible inactivation of the epidermal growth factor receptor and erbB2, by a new class of tyrosine kinase inhibitor. Proc. Natl. Acad. Sci. U.S.A. 95(20): 12022-12027.
26. Bos M, et al. (1997) PD153035, a tyrosine kinase inhibitor, prevents epidermal growth factor receptor activation and inhibits growth of cancer cells in a receptor number-dependent manner. Clin. Cancer Res. 3(11):2099-2106.
27. Hancock CN, et al. (2005) Identification of novel extracellular signal-regulated kinase docking domain inhibitors. J. Med. Chem. 48(14):4586-4595.
28. Ohori M, et al. (2005) Identification of a selective ERK inhibitor and structural determination of the inhibitor-ERK2 complex. Biochem. Biophys. Res. Commun. 336(1):357-363.
29. Gilmartin AG, et al. (2011) GSK1120212 (JTP-74057) Is an Inhibitor of MEK Activity and Activation with Favorable Pharmacokinetic Properties for Sustained In Vivo Pathway Inhibition. Clin. Cancer Res. 17(5):989-1000.
30. Roberts PJ & Der CJ (2007) Targeting the Raf-MEK-ERK mitogen-activated protein kinase cascade for the treatment of cancer. Oncogene 26(22):3291-3310.
31. Favata MF, et al. (1998) Identification of a novel inhibitor of mitogen-activated protein kinase kinase. J. Biol. Chem. 273(29):18623-18632 (1998).
32. Garcia-Martinez JM, et al. (2009) Ku-0063794 is a specific inhibitor of the mammalian target of rapamycin (mTOR). Biochem. J. 421(1):29-42.
33. Liu QS, et al. (2010) Discovery of 1-(4-(4-Propionylpiperazin-1-yl)-3-(trifluoromethyl)phenyl)-9-(quinolin- 3-yl)benzo h 1,6 naphthyridin-2(1H)-one as a Highly Potent, Selective Mammalian Target of Rapamycin (mTOR) Inhibitor for the Treatment of Cancer. J. Med. Chem. 53(19):7146-7155.
34. Pearce LR, et al. (2010) Characterization of PF-4708671, a novel and highly specific inhibitor of p70 ribosomal S6 kinase (S6K1). Biochem. J. 431(2):245-255.
35. Okuzumi T, et al. (2009) Inhibitor hijacking of Akt activation. Nat. Chem. Biol. 5(7):484-493.
36. Raynaud Fl, et al. (2009) Biological properties of potent inhibitors of class I phosphatidylinositide 3-kinases: from PI-103 through PI-540, PI-620 to the oral agent GDC-0941. Mol. Cancer Ther. 8(7):1725-1738.
37. Miller TW, Rexer BN, Garrett JT, & Arteaga CL (2011) Mutations in the phosphatidylinositol 3-kinase pathway: role in tumor progression and therapeutic implications in breast cancer. Breast Cancer Res. 13(6):224.
38. Raynaud Fl, et al. (2007) Pharmacologic characterization of a potent inhibitor of class I phosphatidylinositide 3-kinases. Cancer Res. 67(12):5840-5850.
39. Martinybaron G, et al. (1993) Selective inhibition of protein kinase C isozymes by the indolocarbazole Gö 6976. J. Biol. Chem. 268(13):9194-9197.
40. Toullec D, et al. (1991) The bisindolylmaleimide GF-109203X is a potent and selective inhibitor of protein kinase C. J. Biol. Chem. 266(24):15771-15781.
41. McCluskey A, Sim ATR, & SakoffJA (2002) Serine-threonine protein phosphatase inhibitors: Development of potential therapeutic strategies. J. Med. Chem. 45(6):1151-1175.
42. Li YM & Casida JE (1992) Cantharidin-binding protein - identification as protein phosphatase-2A. Proc. Natl. Acad. Sci. U.S.A. 89(24):11867-11870.
43. Tamura M, et al. (2005) Development of specific Rho-kinase inhibitors and their clinical application. Biochim. Biophys. Acta-Proteins and Proteomics 1754(1-2):245-252.
44. Ishizaki T, et al. (2000) Pharmacological properties of Y-27632, a specific inhibitor of Rho-associated kinases. Mol. Pharmacol. 57(5):976-983.

### Results

We set out to classify phosphorylation sites into groups defined by their patterns of modulation in response to inhibitors of cell signaling. We treated MCF7 cells with single small-molecule inhibitors against a panel of kinases and measured the resulting changes in phosphorylation using MS-based phosphoproteomics (Fig. 1A). Twenty structurally distinct kinase inhibitors, two phosphatase inhibitors, or DMSO vehicle control (Fig. 1B) were the conditions used for the experiments. The kinases targeted in the experiments, alongside the inhibitors used, were chosen on the basis of their known involvement in growth factor and metabolic signaling, and their current therapeutic potential.

The MS experiments resulted in the identification of a total of 13,405 unique phosphopeptide ions across the six analytical replicates per condition (three technical and two biological). A quality control summary of these data is shown in Fig. 5. Each phosphopeptide was quantified across all the experimental conditions using a previously described label-free methodology (18, 19), generating 1,930,320 data points.

**Phosphoproteomics data allow the classification of kinase inhibitors based on the targets they inhibit.** We observed 4,651 phosphorylation sites significantly reduced in abundance by at least one kinase inhibitor (adjusted *P* ≤ 0.05). To assess the global effects of inhibitors on these sites, we used principal component analysis (PCA). This multivariate statistical analysis method allows the separation of experimental conditions based on the overall structure of the underlying data. PCA of the inhibitor-treated phosphoproteomes demonstrated that inhibitors directed against the same kinase were closer to each other in principal component space than to the rest of the inhibitors (Fig. 1C), indicating that inhibitors against the same kinase produced similar effects on global phosphorylation. The only exception to this observation was the ERK inhibitors; these being close in the PC1 but not PC2 dimension, suggesting that these had slightly different quantitative effects on the phosphoproteome. While inhibitors against kinases related to the MAPK signaling cascade (EGFR, MEK, and ERK) separated from those related to the PI3K/mTOR signaling axis (PI3K, mTOR, p70S6K), inhibitors of Akt associated more closely with EGFR and CAMK2 inhibitors than to inhibitors of its well-known upstream activator, PI3K. As would be expected, inhibitors targeting PP2A (a protein phosphatase) separated well from the kinase inhibitors. Analysis using a correlation matrix reinforced the relationships observed between mTOR, Akt and PI3K inhibitors and between EGRF, ERK and MEK inhibitor pairs (Fig. 1D). Statistical significance of each correlation is shown in Fig. 6. Motif analysis (14) further revealed that the inhibitor pairs exhibited strongly correlated impacts on specific phosphorylation motifs whilst mirroring the relationships seen in Fig. 1C and 1D (Fig. 1E). Together, these data show that inhibitors against the same kinases produced more similar changes in the phosphoproteomes than to the rest of inhibitors and, with the exception of Akt, those against the same canonical pathways also affected a common set of phosphorylation sites.

**Deriving activity markers of inhibitor targets from phosphoproteomics data.** To provide an additional level of classification to the dataset, we further grouped the phosphorylation sites based upon their behavior under treatment with inhibitors against the same kinase. The number of phosphorylation sites selected at this stage was dependent on the stringency of the thresholds used for selection (Fig. 7). Most importantly however, we selected only those phosphorylation sites that reached the required statistical thresholds in both inhibitor treatments targeting the same kinase. The selection of phosphorylation sites inhibited by structurally distinct compounds targeting the same kinase should result in datasets enriched in phosphorylation sites specific to the intended kinase. This is illustrated in Fig. 2A for the phosphorylation sites modulated by the two different Akt inhibitors (MK-2206 and Akt Inhibitor VIII), which shows phosphorylation sites inhibited by both inhibitors (darkly shaded data points below the lower horizontal dotted line and left of the leftmost dotted line (FR ≤ -1.0)in Fig. 2A) as well as sites specifically inhibited by one compound but not the other (darkly shaded data points above the lower horizontal dotted line and below the next highest dotted horizontal line; darkly shaded data points right of the leftmost dotted vertical line and left of the second to left dotted vertical line (-0.75 < FR > -1.0) in Fig. 2A). We hypothesized that sites inhibited by the Akt inhibitor MK-2206 but not by Akt inhibitor VIII, and *vice versa,* were off-target effects, whilst those inhibited by both compounds were more likely to be truly downstream of Akt. This analysis was performed for each of the 10 kinases targeted in the study (Fig. 1A) and revealed 610 phosphorylation sites reduced in abundance by at least one inhibitor pair (i.e., by both inhibitors against the same kinase). These sites, although not necessarily directly phosphorylated by the intended target kinase, as they could be phosphorylated by kinases acting downstream or by closely related kinases, are readouts of the actual kinases affected by the inhibitor/compound, and thus we referred to them as compound-target activity markers (CTAMs).

**Inferring signaling network topology from phosphoproteomics data.** Visualizing the 610 identified CTAM phosphorylation sites simultaneously revealed that a large number of them were identified as markers of more than one compound-target pair (Fig. 2B). Therefore, to investigate the relationships between inhibitor pairs further, and in order to allow inference of signaling network topology from the data, the 610 CTAM phosphorylation sites were further classified based on whether these were inhibited by one or more inhibitor pairs. A number of known patterns of kinase signaling topology emerged from this analysis (Fig. 2C). For example, we identified 41 phosphorylation sites that were inhibited by the inhibitor pairs against Akt, mTOR, p70S6K and PI3K (Fig. 2C). Consistent with previous knowledge, these sites included those on BAD, Ser⁴²⁷ on KS6B1 (p70S6K1), and Thr¹¹³⁵ on RICTOR (Fig. 2C) (20-22). Alongside these, sites that have not yet been functionally annotated were also present in this group, and together these 41 sites were classified as members of the Akt-mTOR-PI3K-p70S6K CTAM group. Similarly, sites modulated by Akt, mTOR and PI3K inhibitor pairs, but not by the p70S6K inhibitor pair, included GSK3β at Ser⁹, Myc at Ser⁶², and AKTS1 (also known as PRAS40) at Ser¹⁸³ (Fig. 8); a total of 55 phosphorylation sites were found to have this pattern of inhibition and defined an Akt-mTOR-PI3K group that is independent of p70S6K. In addition to these well-known kinase cascades, we also found evidence for the existence of as yet uncharacterized relationships between the kinases targeted by the inhibitors and/or the inhibitors themselves; examples include sites modulated by mTOR and PI3K inhibitor pairs without the involvement of Akt or p70S6K (37 substrates, Fig. 2B-C). As Fig. 2B-C illustrate, we also found evidence of sites inhibited by both Akt inhibitors but unaffected by PI3K and other inhibitors (284 substrates) and PI3K sites independent of Akt and mTOR (33 substrates). Overall, the 610 phosphorylation site activity markers found in this study (Table 1) were grouped into 55 CTAM groups.

Visualizing the data as a bipartite, undirected network graph (Fig. 2D) further revealed the way in which the investigated kinase inhibitors related to each other in the signaling network. As expected and in concordance with published data and their canonical associations, Akt, PI3K, p70S6K, and mTOR inhibitor pairs affected a large number of common phosphorylation sites and hence grouped together. Similarly, inhibitor pairs targeting the MAPK pathway (EGFR, MEK, and ERK) also grouped together and with CAMK2 (Fig. 2D). Randomization of the network's topology revealed that these associations were not likely to have occurred by chance (Fig. 9). These data therefore show that, although canonical associations between kinases were well represented in our dataset (e.g., EGFR-MEK-ERK and PI3K-Akt-mTOR-p70S6K), the existence of unexpected signaling routes also emerged from these data, including the existence of PI3K-mTOR signaling independent of Akt. Moreover, this analysis demonstrated the high degree of connectivity between these kinases.

**Characterization of the identified CTAM groups' behavior and probing of network plasticity.** We next sought to confirm whether the CTAM groups could be used to measure the biochemical activation of pathways within the network, and thus provide a snapshot of the network's activation status at any given time, under any given condition. We hypothesized that, should these groups provide reliable readouts of network branch activity, each CTAM group should show the expected behavioral changes when the network is perturbed or stimulated under well-characterized experimental conditions. In addition, we reasoned that individual members within each group should demonstrate similar quantitative behavior to one another. We thus monitored the dynamics of phosphorylation of the CTAM groups across cells treated with either EGF or IGF-1 at five independent time-points. A quality control summary for this dataset is shown in Fig. 10.

We observed that the temporal phosphorylation dynamics of CTAM groups commonly associated with EGFR and IGF-1 R signaling (relative to the 0 minute control in each case) were inline with the previously reported effects of EGF and IGF-1 on kinase signaling (Fig. 3A) (23, 24). For example, consistent with the known temporal dynamics of MAPK pathway activation, the EGFR-MEK group underwent significant, acute up-regulation upon stimulation with both growth factors for 5 min, before beginning to decline to a lower level at 60 min (Fig. 3A-B). These data were in agreement with individual MS and western blot data for the canonical EGFR-responsive MAPK (Thr²⁰²/Tyr²⁰⁴) and Akt sites (Ser⁴⁷³) (Fig. 11). The median relative standard deviations (i.e., coefficient of variations) of individual phosphorylation sites within CTAM groups were 0.454 and 0.518 for EGF and IGF-1 respectively (Fig. 3C), thus reflecting that these behaved similarly upon cell stimulation with the two growth factors. Taken together, the data shown in Fig. 3 provide evidence to support the notion that CTAM groups were readouts of the functional activation of branches within the network.

**Analysis of network plasticity in models of acquired resistance to kinase inhibitors.** To further investigate kinase signaling plasticity in our CTAM-defined signaling network, we measured the phosphorylation sites that define the network in cancer cell-line models of acquired resistance to two kinase inhibitors in clinical development; namely, GDC-0941 (a pan class I PI3K inhibitor) and KU-0063794 (an mTORC1/2 inhibitor) (25, 26). We obtained six independent cell cultures resistant to each of the inhibitors when compared to the parental cells from which they were derived (three per drug: MCF7-G and MCF7-K resistant to GDC-041 and KU-0063794, respectively). To achieve this, we chronically exposed the cells to an increasing concentration of the relevant inhibitor up to a maximum of 1 µM. The cells were initially challenged with a low concentration of each drug (100 nM) so as not to bias the resistance selection for intrinsically resistant cells. The resultant cell-lines were able to proliferate in the presence of 1 µM of inhibitor, whereas parental cells were unable to do so under the same conditions (Fig. 4A-B). We quantified the phosphoproteomes of these cells and normalized these measurements to total protein by simultaneously analyzing the total proteome. A summary of the quantitative and qualitative data is shown in Fig. 12.

Analysis of the kinase network in the presence of inhibitor revealed that the large majority of the CTAM groups involving mTOR were down-regulated in all of the mTOR inhibitor resistant (MCF7-K) cell-lines (green and orange arrows in Fig. 4C). Similarly, CTAM groups containing PI3K were down-regulated in all of the PI3K inhibitor resistant (MCF7-G) cell-lines (green arrows in Fig. 4C). We confirmed these data by measuring well-known markers of pathway activities, which showed that our results were consistent with the levels of key regulatory phosphorylation sites governing these pathways on Akt (Ser⁴⁷³) and p70S6K (Thr³⁸⁹), as determined by western blot (Fig. 13). These data suggested that the pathways targeted by the inhibitors remained inhibited in resistant cells in the presence of the drug. We therefore reasoned that resistance was not the result of differences in how resistant cells metabolized the inhibitors and that instead, consistent with other studies (27), resistance more likely arose as a consequence of a rewiring of kinase signaling. Of interest, this rewiring was markedly dissimilar between the MCF7-K and MCF7-G cell-lines (Fig. 4C), suggesting that the resistance mechanisms that had evolved against the mTOR inhibitor were distinct to those evolved in response to the PI3K inhibitor. This hypothesis was reinforced through the use of an unbiased multivariate analysis of the normalized phosphoproteomics data, which highlighted the differences between the parental and resistant cells, and the differences between the rewiring of MCF7-K and MCF7-G cells, as these separated clearly in principal component space (Fig 4D). Unexpectedly, however, cells resistant to the same inhibitor also separated in PC space, suggesting that the activation state of the signaling network was heterogeneous between individual resistant lines (Fig. 4C-D) despite these being derived from the same parental culture, at the same passage number, and being exposed to identical experimental conditions for the same amount of time.

Since the resistant cell-lines seemed to differ in the way in which they had rewired their signaling network compared to parental cells and to each other (Fig. 4C-D), we hypothesized that each cell-line should respond differently to a panel of small-molecule kinase inhibitors alone and in combination, as their response would be a function of their signaling network's activity. To test this prediction, we treated each of the cell-lines with a panel of small-molecule inhibitors (targeting PI3K, mTOR, CAMK2, Akt, MEK, and EGFR) and measured their relative cell viability using the MTS and crystal violet assays (Fig. 14A and B, respectively). These inhibitors were chosen because the CTAMs of their associated kinases were increased in abundance in some of the resistant cells relative to parental (Fig. 4C), thus suggesting that these kinases may be involved in the resistance phenotype. An unbiased, multivariate analysis of the resulting data revealed that the resistant and parental cells responded differently to the inhibitors, as they separated in PC space. Moreover, this analysis separated the MCF7-G and -K cells and the individual resistant cell-lines from one-another in a manner reflecting that observed in the PCA of the phosphoproteomics data (Fig. 4E-F). Taken together, these data indicate that the heterogeneous rewiring of the signaling network in resistant cells observed by CTAM analysis (Fig. 4C) resulted in functional differences in how cells responded to perturbations to the network (Fig. 4E-F).

### Discussion

In this study, we first performed a thorough analysis of the connections that exist between the nodes of the PI3K-MEK kinase network (Figs 1 and 2). This initial study revealed both expected and unexpected links between kinases, signaling pathways and the pharmacological agents targeting them. For example, the known PI3K-Akt-mTOR-p70S6K, PI3K-Akt-mTOR and MEK-ERK relationships were well represented in our data; however, we also found evidence for as yet uncharacterized connections between kinase inhibitor targets, such as those defined by mTOR-PI3K associations without the involvement of Akt and Akt inhibitor-dependent but PI3K inhibitor-independent sites. Overall our data exemplify the complex relationship between kinases in signaling networks and illustrate that our knowledge of this complexity is still very limited.

An advantage of defining signaling routes using a set of experimental data derived from a defined system - in contrast to approaches that compile information from the literature (28, 29) - is that cell signaling events are often cell-type and -context dependent (30). Therefore, 'averaged' signaling networks, derived from disparate cell types and organisms, as shown in canonical signaling pathway schematics, are not always representative of how signaling networks are in-fact wired in specific cellular systems. Although efforts have been made to overlay empirical transcriptional data onto these averaged networks (31), a key aspect of our study is that we not only provide evidence of as yet uncharacterized signaling routes but also identified phosphorylation sites markers of such routes' activities specific to our cell-line model, which could then be used to measure the dynamics and circuitry of the kinase network in a systematic manner. The CTAM approach to define signaling network branches, which can then be measured in subsequent experiments, has conceptual similarities to approaches that derive cell biological information from gene expression patterns by examining how such patterns correlate with compendia of profiles obtained from systematic gene inactivation experiments (32). The observation that CTAM groups were modulated by growth factors with the expected kinetics [(23, 24, Fig. 3] and that these changes were similar for members of such groups (Fig. 3) provided evidence to suggest that these CTAM groups are biochemical readouts of signaling activity.

Signaling networks are not static, but rather highly dynamic structures that are extremely plastic in response to external stimuli. A comparison of the network between parental and cells resistant to either a PI3K or mTORC1/2 inhibitor revealed widespread differences in CTAM group abundances in three separate resistant cell cultures per inhibitor (Fig. 4C). Our data are therefore consistent with published studies showing that kinase signaling is remodeled in response to chronic kinase inhibition (33, 34). Due to the depth of our analyses, however, our data emphasize the extent to which signaling networks are modulated as a whole in order to overcome chronic inhibition of single nodes. Concurrent with these observations, comparison of the data on network status (phosphoproteomics) and phenotypes (responses to inhibitors) for each cell-line using multivariate analyses indicated that the activity of the signaling network was a reflection of the cells' newly acquired phenotypes. This was evidenced in the PCAs of all these disparate sets of data, which separated the resistant from parental cells, and the MCF7-K from the MCF7-G cells in a similar manner (Fig. 4D-F). Our data therefore accentuate the complex relationship between PI3K and mTORC1/2, as the cells treated with the two inhibitors changed their signaling differently thus suggesting different mechanisms of resistance in response to the two inhibitors (Fig. 4D-F). This was unexpected as PI3K and mTOR are often placed in the same canonical signaling pathway. However, these data are consistent with the observation that phosphorylation sites modulated by mTORC1/2 or PI3K inhibitors only partially overlapped (Fig. 2E), and with a recent study highlighting mTOR's independence from PI3K in some systems (35). The simple maintenance of cells in culture could have contributed to the evolution of cell networks shown in Fig. 4; however, the observation that chronic exposure to mTORC1/2 or PI3K inhibitors produced marked differences in network remodeling argues that drug exposure had a greater effect on how signaling evolved than that which would be observed as a result of long-term culture without the application of such a selective pressure.

Recent publications indicate that, rather than there being a single mechanism of acquired resistance for a given therapy, there is a plethora of ways in which signaling networks can be rewired in cancer cells as these become resistant to targeted therapies (36, 37). It is not known, however, whether the way in which cancer cells develop resistance is predetermined by the molecular imprinting of cancer cells at presentation (i.e, before the resistance phenotype ensues). This understanding has potential implications for cancer therapeutics as, if the evolutionary pathways that lead to the acquisition of resistance were reliant on genomic or other molecular factors present in tumor cells at presentation, then analyzing the initial conditions of cancer cells could, at least in principle, be used to predict the resistance mechanism more likely to occur in such a tumor.

Contrary to this idea, our data suggest that it may not be possible to predict the particular mechanism of resistance of a given tumor by analyzing it at the molecular level at presentation. Indeed, in our study, the same cell-line was split into six identical populations, which were then maintained in the same concentrations of inhibitors by the same operator for the same amount of time and passage numbers. Unexpectedly, the CTAM network profiles were highly heterogeneous across cells resistant to the same compound (Fig. 4C-D). This heterogeneity in network status had a functional consequence in that cells resistant to the same compound responded differently to inhibitors of signaling at the level of cell proliferation (Fig. 4E-F). Intra-tumoral heterogeneity and evolutionary divergence of initially clonal systems has been documented at the genetic level in both mammalian and bacterial systems (38, 39). Although we cannot entirely exclude the impact of long-term cell culture, our study suggests that chronic treatment with targeted kinase inhibitors profoundly influences the divergence of kinase network signaling. Thus, as identical experimental conditions can result in the evolution of distinct signaling networks (Fig. 4C-D) and drug-resistance phenotypes (Fig. 4E-F), perhaps due to stochastic effects, the initial conditions of the system may not be accurate predictors of the evolutionary route that may lead to resistance. Therefore, if the acquisition of resistance is truly indeterministic and cannot be predicted at disease onset, identification of the resistance mechanisms relevant to each individual patient will most likely require the ability to measure the signaling network in individual tumors after resistance has ensued with depth and without a preconception of how signaling may have been rewired as a result of therapy.

### References (these apply to the Example except for the Detailed Materials & Methods section, above)

1. Du W & Elemento O (2014) Cancer systems biology: embracing complexity to develop better anticancer therapeutic strategies. Oncogene [Epub ahead of print].
2. Papin JA, Hunter T, Palsson BO, & Subramaniam S (2005) Reconstruction of cellular signaling networks and analysis of their properties. Nat. Rev. Mol. Cell Biol. 6(2):99-111.
3. Bodenmiller B, et al. (2010) Phosphoproteomic Analysis Reveals Interconnected System-Wide Responses to Perturbations of Kinases and Phosphatases in Yeast. Sci. Signal 3(153):rs4.
4. Jorgensen C & Linding R (2010) Simplistic pathways or complex networks? Curr. Opin. Genet. Dev. 20(1):15-22.
5. Hsu PP, et al. (2011) The mTOR-regulated phosphoproteome reveals a mechanism of mTORC1-mediated inhibition of growth factor signaling. Science 332(6035):1317-1322.
6. Goltsov A, et al. (2012) Features of the reversible sensitivity-resistance transition in PI3K/PTEN/AKT signaling network after HER2 inhibition. Cell. Signal. 24(2):493-504.
7. Goltsov A, et al. (2011) Compensatory effects in the Pl3K/PTEN/AKT signaling network following receptor tyrosine kinase inhibition. Cell. Signal. 23(2):407-416.
8. Kholodenko BN, et al. (2002) Untangling the wires: a strategy to trace functional interactions in signaling and gene networks. Proc. Natl. Acad. Sci. U.S.A. 99(20):12841-12846.
9. Mukherjee S & Speed TP (2008) Network inference using informative priors. Proc. Natl. Acad. Sci. U.S.A. 105(38):14313-14318.
10. Prill RJ, Saez-Rodriguez J, Alexopoulos LG, Sorger PK, & Stolovitzky G (2011) Crowdsourcing network inference: the DREAM predictive signaling network challenge. Sci. Signal 4(189):mr7.
11. Linding R, et al. (2007) Systematic Discovery of In Vivo Phosphorylation Networks. Cell 129(7):1415-1426.
12. Carlson SM, et al. (2011) Large-Scale Discovery of ERK2 Substrates Identifies ERK-Mediated Transcriptional Regulation by ETV3. Sci. Signal. 4(196).
13. Bensimon A, Heck AJ, & Aebersold R (2012) Mass spectrometry-based proteomics and network biology. Annu. Rev. Biochem. 81:379-405.
14. Casado P, et al. (2013) Kinase-substrate enrichment analysis provides insights into the heterogeneity of signaling pathway activation in leukemia cells. Sci. Signal. 6(268):rs6.
15. Posch C, et al. (2013) Combined targeting of MEK and PI3K/mTOR effector pathways is necessary to effectively inhibit NRAS mutant melanoma in vitro and in vivo. Proc. Natl. Acad. Sci. U.S.A. 110(10):4015-4020.
16. Renshaw J, et al. (2013) Dual blockade of the PI3K/AKT/mTOR (AZD8055) and RAS/MEK/ERK (AZD6244) pathways synergistically inhibits rhabdomyosarcoma cell growth in vitro and in vivo. Clin. Cancer Res. 19(21):5940-5951.
17. Roberts PJ, et al. (2012) Combined PI3K/mTOR and MEK inhibition provides broad antitumor activity in faithful murine cancer models. Clin. Cancer Res. 18(19):5290-5303.
18. Casado P, et al. (2013) Phosphoproteomics data classify hematological cancer cell lines according to tumor type and sensitivity to kinase inhibitors. Genome Biol. 14(4):R37.
19. Montoya A, Beltran L, Casado P, Rodriguez-Prados JC, & Cutillas PR (2011) Characterization of a TiO(2) enrichment method for label-free quantitative phosphoproteomics. Methods 54(4):370-378.
20. Sarbassov DD, et al. (2004) Rictor, a novel binding partner of mTOR, defines a rapamycin-insensitive and raptor-independent pathway that regulates the cytoskeleton. Curr. Biol. 14(14):1296-1302.
21. Hay N & Sonenberg N (2004) Upstream and downstream of mTOR. Genes Dev. 18(16):1926-1945.
22. Kumar JK, Ping RYS, Teong HF, Goh S, & Clement M-V (2011) Activation of a non-genomic Pim-1/Bad-Pser75 module is required for an efficient pro-survival effect of Bcl-xL induced by androgen in LNCaP cells. Int. J. Biochem. Cell. Biol. 43(4):594-603.
23. Vincent AM & Feldman EL (2002) Control of cell survival by IGF signaling pathways. Growth Horm. IGF Res. 12(4):193-197.
24. Ciaccio MF, Wagner JP, Chuu C-P, Lauffenburger DA, & Jones RB (2010) Systems analysis of EGF receptor signaling dynamics with microwestern arrays. Nat. Methods 7(2):148-U195.
25. Garcia-Martinez JM, et al. (2009) Ku-0063794 is a specific inhibitor of the mammalian target of rapamycin (mTOR). Biochem. J. 421(1):29-42.
26. Munugalavadla VM, S Slaga, D Du, C Berry, L Del Rosario, G Yan, Y Boe, M Sun, L Friedman, L S Chesi, M Leif Bergsagel, P Ebens, A (2013) The PI3K inhibitor GDC-0941 combines with existing clinical regimens for superior activity in multiple myeloma. Oncogene 33(3):316-325.
27. Liu P, et al. (2011) Oncogenic PIK3CA-driven mammary tumors frequently recur via PI3K pathway-dependent and PI3K pathway-independent mechanisms. Nat. Med. 17(9):1116-1120.
28. Oda K, Matsuoka Y, Funahashi A, & Kitano H (2005) A comprehensive pathway map of epidermal growth factor receptor signaling. Mol. Sys. Biol. 1:2005.0010.
29. Kanehisa M, Goto S, Sato Y, Furumichi M, & Tanabe M (2012) KEGG for integration and interpretation of large-scale molecular data sets. Nucleic Acids Res. 40(Database issue):D109-114.
30. Alcolea MP, Casado P, Rodriguez-Prados JC, Vanhaesebroeck B, & Cutillas PR (2012) Phosphoproteomic Analysis of Leukemia Cells under Basal and Drug-treated Conditions Identifies Markers of Kinase Pathway Activation and Mechanisms of Resistance. Mol. Cell. Proteomics 11(8):453-466.
31. Dutta B, et al. (2012) A network-based, integrative study to identify core biological pathways that drive breast cancer clinical subtypes. Br. J. Cancer 106(6):1107-1116.
32. Hughes TR, et al. (2000) Functional discovery via a compendium of expression profiles. Cell 102(1):109-126
33. Muranen T, et al. (2012) Inhibition of PI3K/mTOR leads to adaptive resistance in matrix-attached cancer cells. Cancer Cell 21(2):227-239.
34. Liu P, et al. (2011) Oncogenic PIK3CA-driven mammary tumors frequently recur via PI3K pathway-dependent and PI3K pathway-independent mechanisms. Nat. Med. 17(9):1116-U1122.
35. Elkabets M, et al. (2013) mTORC1 Inhibition Is Required for Sensitivity to PI3K p110 alpha Inhibitors in PIK3CA-Mutant Breast Cancer. Sci. Transl. Med. 5(196):196ra99.
36. Klempner SJ, Myers AP, & Cantley LC (2013) What a Tangled Web We Weave: Emerging Resistance Mechanisms to Inhibition of the Phosphoinositide 3-Kinase Pathway. Cancer Disc. 3(12):1345-1354.
37. Lito P, Rosen N, & Solit DB (2013) Tumor adaptation and resistance to RAF inhibitors. Nat. Med. 19(11):1401-1409.
38. Gerlinger M, et al. (2012) Intratumor heterogeneity and branched evolution revealed by multiregion sequencing. N Eng. J. Med. 366(10):883-892.
39. Le Gac M, Plucain J, Hindré T, Lenski RE, & Schneider D (2012) Ecological and evolutionary dynamics of coexisting lineages during a long-term experiment with Escherichia coli. Proc. Natl. Acad. Sci. U.S.A. 109(24):9487-9492.
40. Bolstad BM, Irizarry RA, Astrand M, & Speed TP (2003) A comparison of normalization methods for high density oligonucleotide array data based on variance and bias. Bioinformatics 19(2):185-193.
41. Smyth GK (2004) Linear models and empirical bayes methods for assessing differential expression in microarray experiments. Stat. Appl. Genet. Molec. Biol. 3(1):Article3.
42. Smyth GK (2005) Limma: linear models for microarray data (Springer New York).
43. R Core Team (2013) R: A language and environment for statistical computing (R Foundation for Statistical Computing).

## Claims

1. An *in vitro* method of creating a dataset of modification sites which are affected in the same way by modulators of the same protein modifying enzyme, and are therefore indicative of the activity of the protein modifying enzyme that is affected by the modulators, comprising identifying and/or quantifying modification sites on modified peptides in a first sample which has been treated with a first modulator of a protein modifying enzyme and a second sample which has been treated with a second modulator of the same protein modifying enzyme using mass spectrometry (MS); and grouping modification sites on modified peptides from the first sample which has been treated with the first modulator of a protein modifying enzyme and modification sites on modified peptides from the second sample which has been treated with the second modulator of the same protein modifying enzyme into a single group, according to the effect of said first and second modulators of said protein modifying enzyme on said modification sites, wherein the modification sites on modified peptides from a first sample and modification sites on modified peptides from a second sample are placed into a single group based on the modification sites being affected in the same way by the first and second modulators of the protein modifying enzyme, and wherein said first and second modulators of said protein modifying enzyme are different.

2. The method according to claim 1, wherein said modification sites are selected from the group consisting of phosphorylation sites, acetylation sites, glycosylation sites, methylation sites and lipidation sites.

3. The method according to claim 1 or 2, wherein said first sample and said second sample is from either:
(a) a bodily fluid, optionally urine or blood; or
(b) a tissue, optionally cancer tissue, optionally a tumor.

4. The method according to any one of claims 1 to 3, wherein said protein modifying enzyme is selected from the group consisting of a protein kinase, protein phosphatase, protein glycosyltransferase, protein acetyltransferase, protein methyltransferase and protein palmitoyltransferase.

5. The method according to any one of the preceding claims, wherein said modulator of a protein modifying enzyme is
(a) a small molecule, RNAi, therapeutic peptide, or antibody; and/or
(b) an inhibitor of a protein modifying enzyme.

6. The method according to claim 5, wherein said inhibitor of a protein modifying enzyme is a kinase inhibitor.

7. The method according to claim 6, wherein said kinase inhibitor is an inhibitor of a kinase selected from the group consisting of Akt, CAMK2, EGFR, ERK, MEK, mTOR, p70S6K, PI3K, PKC, and ROCK.

8. The method according to any one of the preceding claims, wherein said effect of said first and second modulators of said protein modifying enzyme on said modification site is a reduction or increase in abundance of said modification site compared to a control sample.

9. A method of preparing a database, comprising creating a dataset of modification sites according to a method of any one of the preceding claims, and compiling said dataset into a database.

10. The method according to claim 9, wherein said database further comprises information on the identities of proteins containing the modification sites, the type of modification, the type of sample in which the modification site is present and/or the modulator that increases or decreases the modification at the modification site.

11. An *in vitro* method for diagnosing a disease comprising:
(a) Creating a test dataset of modification sites by:
i. Treating one or more test samples from a subject with one or more modulators of a protein modifying enzyme,
ii. Identifying and/or quantifying modification sites on modified peptides in the one or more test samples,
iii. Grouping modification sites on modified peptides from the one or more test samples into a single group, thereby creating the test dataset of modification sites, and
iv. optionally compiling the test dataset into a test database,
(b) Comparing the test dataset with a dataset of modification sites created according to the method of any one of claims 1 to 8 or comparing the test database a database prepared according to claims 9 or 10,
(c) Finding a significant difference between the test dataset or test database and the control dataset or control database,
(d) Diagnosing that the subject has the disease if a significant difference between the dataset or database is found in step (c).

12. The method of claim 11, wherein the disease is cancer.

13. A method of identifying one or more biomarkers for the activity of a protein modifying enzyme comprising creating a dataset of modification sites according to the method of any one of claims 1 to 8 or preparing a database according to claims 9 or 10, wherein the modification sites in the single group are identified as the one or more biomarkers for the activity of a protein modifying enzyme.

14. An *in vitro* method of determining that a test substance is a modulator of a protein modifying enzyme comprising treating a test sample with the test substance, identifying and/or quantifying modification sites on modified peptides in the test sample, creating a test dataset of modification sites from the modification sites identified and/or quantified in the test sample, further comprising:
(a) Comparing the test dataset with a dataset of modification sites created according to the method of any one of claims 1 to 8 and/or
(b) Compiling said test dataset into a test database and comparing the test database with a database prepared according to claims 9 or 10.

## Patentansprüche

1. In-vitro-Verfahren zum Erzeugen eines Datensatzes von Modifikationsstellen, die von Modulatoren des gleichen proteinmodifizierenden Enzyms auf dieselbe Weise beeinflusst werden und daher die Aktivität des proteinmodifizierenden Enzyms, das von den Modulatoren betroffen ist, anzeigen, umfassend das Identifizieren und/oder Quantifizieren von Modifikationsstellen auf modifizierten Peptiden in einer ersten Probe, die mit einem ersten Modulator eines proteinmodifizierenden Enzyms behandelt wurde, und einer zweiten Probe, die mit einem zweiten Modulator des gleichen proteinmodifizierenden Enzyms behandelt wurde, unter Verwendung von Massenspektrometrie (MS); und das Gruppieren von Modifikationsstellen auf modifizierten Peptiden aus der ersten Probe, die mit dem ersten Modulator eines proteinmodifizierenden Enzyms behandelt wurde, und von Modifikationsstellen auf modifizierten Peptiden aus der zweiten Probe, die mit dem zweiten Modulator des gleichen proteinmodifizierenden Enzyms behandelt wurde, in eine einzelne Gruppe gemäß dem Effekt des besagten ersten und zweiten Modulators des proteinmodifizierenden Enzyms auf die Modifikationsstellen, wobei die Modifikationsstellen auf modifizierten Peptiden aus einer ersten Probe und die Modifikationsstellen auf modifizierten Peptiden aus einer zweiten Probe basierend darauf, dass die Modifikationsstellen auf dieselbe Weise von dem ersten und dem zweiten Modulator des proteinmodifizierenden Enzyms beeinflusst werden, in eine einzelne Gruppe platziert werden und wobei der besagte erste und der besagte zweite Modulator des proteinmodifizierenden Enzyms unterschiedlich sind.

2. Verfahren nach Anspruch 1, wobei die besagten Modifikationsstellen aus der Gruppe bestehend aus Phosphorylierungsstellen, Acetylierungsstellen, Glykosylierungsstellen, Methylierungsstellen und Lipidierungsstellen ausgewählt sind.

3. Verfahren nach Anspruch 1 oder 2, wobei die besagte erste Probe und die besagte zweite Probe entweder aus:
(a) einer Körperflüssigkeit, gegebenenfalls Urin oder Blut; oder
(b) einem Gewebe, gegebenenfalls Krebsgewebe, gegebenenfalls einem Tumor, stammen.

4. Verfahren nach einem der Ansprüche 1 bis 3, wobei das besagte proteinmodifizierende Enzym aus der Gruppe bestehend aus einer Proteinkinase, Proteinphosphatase, Proteinglycosyltransferase, Proteinacetyltransferase, Proteinmethyltransferase und Proteinpalmitoyltransferase ausgewählt ist.

5. Verfahren nach einem der vorhergehenden Ansprüche, wobei der besagte Modulator eines proteinmodifizierenden Enzyms
(a) ein kleines Molekül, eine RNAi, ein therapeutisches Peptid oder ein Antikörper; und/oder
(b) ein Inhibitor eines proteinmodifizierenden Enzyms ist.

6. Verfahren nach Anspruch 5, wobei der besagte Inhibitor eines proteinmodifizierenden Enzyms ein Kinaseinhibitor ist.

7. Verfahren nach Anspruch 6, wobei der besagte Kinaseinhibitor ein Inhibitor einer Kinase ist, ausgewählt aus der Gruppe bestehend aus Akt, CAMK2, EGFR, ERK, MEK, mTOR, p70S6K, PI3K, PKC und ROCK.

8. Verfahren nach einem der vorhergehenden Ansprüche, wobei der besagte Effekt des besagten ersten und zweiten Modulators des proteinmodifizierenden Enzyms auf die besagte Modifikationsstelle in einer Verringerung oder Erhöhung der Häufigkeit der Modifikationsstelle im Vergleich zu einer Kontrollprobe besteht.

9. Verfahren zum Erstellen einer Datenbank, umfassend das Erzeugen eines Datensatzes von Modifikationsstellen gemäß einem Verfahren nach einem beliebigen der vorhergehenden Ansprüche und das Kompilieren des besagten Datensatzes zu einer Datenbank.

10. Verfahren nach Anspruch 9, wobei die besagte Datenbank ferner Informationen über die Identitäten von Proteinen, die die Modifikationsstellen enthalten, den Typ der Modifikation, den Typ der Probe, in der die Modifikationsstelle vorhanden ist, und/oder den Modulator, der die Modifikation an der Modifikationsstelle erhöht oder verringert, umfasst.

11. In-vitro-Verfahren zum Diagnostizieren einer Krankheit, umfassend:
(a) Erzeugen eines Testdatensatzes von Modifikationsstellen durch:
i. Behandeln einer oder mehrerer Testproben aus einem Subjekt mit einem oder mehreren Modulatoren eines proteinmodifizierenden Enzyms,
ii. Identifizieren und/oder Quantifizieren von Modifikationsstellen auf modifizierten Peptiden in der einen oder den mehreren Testproben,
iii. Gruppieren von Modifikationsstellen auf modifizierten Peptiden aus der einen oder den mehreren Testproben in eine einzelne Gruppe, wodurch der Testdatensatz der Modifikationsstellen erzeugt wird, und
iv. gegebenenfalls Kompilieren des Testdatensatzes zu einer Testdatenbank,
(b) Vergleichen des Testdatensatzes mit einem Datensatz von Modifikationsstellen, der gemäß dem Verfahren eines der Ansprüche 1 bis 8 erzeugt wurde, oder Vergleichen der Testdatenbank mit einer Datenbank, die gemäß den Ansprüchen 9 oder 10 erstellt wurde;
(c) Finden eines signifikanten Unterschieds zwischen dem Testdatensatz oder der Testdatenbank und dem Kontrolldatensatz oder der Kontrolldatenbank,
(d) Diagnostizieren, dass das Subjekt an der Krankheit leidet, wenn in Schritt (c) ein signifikanter Unterschied zwischen dem Datensatz oder der Datenbank gefunden wird.

12. Verfahren von Anspruch 11, wobei es sich bei der Krankheit um Krebs handelt.

13. Verfahren zur Identifizierung eines oder mehrerer Biomarker für die Aktivität eines proteinmodifizierenden Enzyms, umfassend das Erzeugen eines Datensatzes von Modifikationsstellen gemäß dem Verfahren nach einem der Ansprüche 1 bis 8 oder das Erstellen einer Datenbank gemäß den Ansprüchen 9 oder 10, wobei die Modifikationsstellen in der einzelnen Gruppe als der eine oder die mehreren Biomarker für die Aktivität eines proteinmodifizierenden Enzyms identifiziert werden.

14. In-vitro-Verfahren zur Bestimmung, dass eine Testsubstanz ein Modulator eines proteinmodifizierenden Enzyms ist, umfassend das Behandeln einer Testprobe mit der Testsubstanz, das Identifizieren und/oder Quantifizieren von Modifikationsstellen auf modifizierten Peptiden in der Testprobe, das Erzeugen eines Testdatensatzes von Modifikationsstellen aus den in der Testprobe identifizierten und/oder quantifizierten Modifikationsstellen, ferner umfassend:
(a) Vergleichen des Testdatensatzes mit einem Datensatz von Modifikationsstellen, der gemäß dem Verfahren eines der Ansprüche 1 bis 8 erzeugt wurde, und/oder
(b) Kompilieren des Testdatensatzes zu einer Testdatenbank und Vergleichen der Testdatenbank mit einer Datenbank, die gemäß den Ansprüchen 9 oder 10 erstellt wurde.

## Revendications

1. Procédé *in vitro* de création d'un ensemble de données de sites de modification qui sont affectés de la même façon par des modulateurs de la même enzyme de modification de protéine, et sont donc indicatives de l'activité de l'enzyme de modification de protéine qui est affectée par les modulateurs, comprenant l'identification et/ou la quantification de sites de modification sur des peptides modifiés dans un premier échantillon qui a été traité avec un premier modulateur d'une enzyme de modification de protéine et un deuxième échantillon qui a été traité avec un deuxième modulateur de la même enzyme de modification de protéine au moyen d'une spectrométrie de masse (MS) ; et le regroupement de sites de modification sur des peptides modifiés du premier échantillon qui a été traité avec le premier modulateur d'une enzyme de modification de protéine et de sites de modification sur des peptides modifiés du deuxième échantillon qui a été traité avec le deuxième modulateur de la même enzyme de modification de protéine dans un groupe unique, selon l'effet desdits premier et deuxième modulateurs de ladite enzyme de modification de protéine sur lesdits sites de modification, dans lequel les sites de modification sur des peptides modifiés d'un premier échantillon et les sites de modification sur des peptides modifiés d'un deuxième échantillon sont placés dans un groupe unique sur la base des sites de modification étant affectés de la même façon par les premier et deuxième modulateurs de l'enzyme de modification de protéine, et dans lequel lesdits premier et deuxième modulateurs de ladite enzyme de modification de protéine sont différents.

2. Procédé selon la revendication 1, dans lequel lesdits sites de modification sont choisis dans le groupe constitué de sites de phosphorylation, sites d'acétylation, sites de glycosylation, sites de méthylation et sites de lipidation.

3. Procédé selon la revendication 1 ou 2, dans lequel ledit premier échantillon et ledit deuxième échantillon provient de :
(a) un fluide corporel, facultativement de l'urine ou du sang ; ou
(b) un tissu, facultativement un tissu cancéreux, facultativement une tumeur.

4. Procédé selon l'une quelconque des revendications 1 à 3, dans lequel ladite enzyme de modification de protéine est choisie dans le groupe constitué d'une protéine kinase, une protéine phosphatase, une protéine glycosyltransférase, une protéine acétyltransférase, une protéine méthyltransférase et une protéine palmitoyltransférase.

5. Procédé selon l'une quelconque des revendications précédentes, dans lequel ledit modulateur d'une enzyme de modification de protéine est
(a) une petite molécule, un ARNi, un peptide thérapeutique ou un anticorps ; et/ou
(b) un inhibiteur d'une enzyme de modification de protéine.

6. Procédé selon la revendication 5, dans lequel ledit inhibiteur d'une enzyme de modification de protéine est un inhibiteur de kinase.

7. Procédé selon la revendication 6, dans lequel ledit inhibiteur de kinase est un inhibiteur d'une kinase choisie dans le groupe constitué d'Akt, CAMK2, EGFR, ERK, MEK, mTOR, p70S6K, PI3K, PKC et ROCK.

8. Procédé selon l'une quelconque des revendications précédentes, dans lequel ledit effet desdits premier et deuxième modulateurs de ladite enzyme de modification de protéine sur ledit site de modification est une réduction ou une augmentation de l'abondance dudit site de modification par rapport à un échantillon témoin.

9. Procédé de préparation d'une base de données, comprenant la création d'un ensemble de données de sites de modification selon un procédé de l'une quelconque des revendications précédentes, et la compilation dudit ensemble de données dans une base de données.

10. Procédé selon la revendication 9, dans lequel ladite base de données comprend en outre des informations sur les identités de protéines contenant les sites de modification, le type de modification, le type d'échantillon dans lequel le site de modification est présent et/ou le modulateur qui augmente ou diminue la modification au site de modification.

11. Procédé *in vitro* pour diagnostiquer une maladie comprenant :
(a) la création d'un ensemble de données d'essai de sites de modification par :
i. traitement d'un ou plusieurs échantillons d'essai d'un sujet avec un ou plusieurs modulateurs d'une enzyme de modification de protéine,
ii. identification et/ou quantification de sites de modification sur des peptides modifiés dans les un ou plusieurs échantillons d'essai,
iii. regroupement de sites de modification sur des peptides modifiés des un ou plusieurs échantillons d'essai dans un groupe unique, de façon à créer l'ensemble de données d'essai de sites de modification, et
iv. facultativement, compilation de l'ensemble de données d'essai dans une base de données d'essai,
(b) la comparaison de l'ensemble de données d'essai avec un ensemble de données de sites de modification créé selon le procédé de l'une quelconque des revendications 1 à 8 ou la comparaison de la base de données d'essai à une base de données préparée selon les revendications 9 ou 10,
(c) la détermination d'une différence significative entre l'ensemble de données d'essai ou la base de données d'essai et l'ensemble de données témoin ou la base de données témoin,
(d) le diagnostic du fait que le sujet a la maladie si une différence significative entre les ensembles de données ou les bases de données est observée dans l'étape (c) .

12. Procédé selon la revendication 11, dans lequel la maladie est un cancer.

13. Procédé d'identification d'un ou plusieurs biomarqueurs pour l'activité d'une enzyme de modification de protéine comprenant la création d'un ensemble de données de sites de modification selon le procédé de l'une quelconque des revendications 1 à 8 ou la préparation d'une base de données selon les revendications 9 ou 10, dans lequel les sites de modification dans le groupe unique sont identifiés comme étant les un ou plusieurs biomarqueurs pour l'activité d'une enzyme de modification de protéine.

14. Procédé *in vitro* de détermination du fait qu'une substance d'essai est un modulateur d'une enzyme de modification de protéine comprenant le traitement d'un échantillon d'essai avec la substance d'essai, l'identification et/ou la quantification de sites de modification sur des peptides modifiés dans l'échantillon d'essai, la création d'un ensemble de données d'essai de sites de modification à partir des sites de modification identifiés et/ou quantifiés dans l'échantillon d'essai, comprenant en outre :
(a) la comparaison de l'ensemble de données d'essai à un ensemble de données de sites de modification créé selon le procédé selon l'une quelconque des revendications 1 à 8 et/ou
(b) la compilation dudit ensemble de données d'essai dans une base de données d'essai et la comparaison de la base de données d'essai à une base de données préparée selon les revendications 9 ou 10.
